# EUROPEAN PATENT SPECIFICATION

(11) **EP 3 436 010 B1**
(45) Date of publication and mention of the grant of the patent: **10.03.2021**
(21) Application number: 17719380.2
(22) Date of filing: 28.03.2017
(51) Int. Cl.: A61K 31/445, A61K 31/4468, A61K 45/06, A61K 31/00, G01N 33/68, A61P 25/28

(54) **5-HT2A SEROTONIN RECEPTOR INVERSE AGONISTS OR ANTAGONISTS FOR USE IN REDUCING AMYLOID-BETA PEPTIDES AND ACCUMULATION OF AMYLOID PLAQUES**
INVERSE 5-HT2A-SEROTONIN-REZEPTOR-AGONISTEN ODER -ANTAGONISTEN ZUR VERWENDUNG IN DER VERRINGERUNG VON AMYLOID-BETA-PEPTIDEN UND IN DER VERRINGERUNG DER AKKUMULATION VON AMYLOID-PLAQUES
AGONISTES OU ANTAGONISTES INVERSES DU RÉCEPTEUR DE LA SÉROTONINE 5-HT2A DESTINÉS À ÊTRE UTILISÉS DANS LE BUT DE RÉDUIRE LES PEPTIDES BÈTA-AMYLOÏDES ET L'ACCUMULATION DE PLAQUES AMYLOÏDES

(30) Priority: 29.03.2016 US 201662314857 P; 04.04.2016 SE 1630067
(43) Date of publication of application: 06.02.2019
(73) Proprietor: Acadia Pharmaceuticals Inc., San Diego, CA 92130 (US)
(72) Inventor: BURSTEIN, Ethan, San Diego, CA 92130 (US)
(74) Representative: Viering, Jentschura & Partner mbB Patent- und Rechtsanwälte
(86) International application number: PCT/US2017/024526
(87) International publication number: WO 2017/172757

(56) References cited:
- EP-A1- 1 576 985
- WO-A2-01/89498
- WO-A2-02/38142
- WO-A2-2007/133802
- WO-A2-2009/039460
- WO-A2-2017/011767
- JOHN E. MORLEY ET AL: "Antibody to Amyloid beta Protein Alleviates Impaired Acquisition, Retention, and Memory Processing in SAMP8 Mice", NEUROBIOLOGY OF LEARNING AND MEMORY., vol. 78, no. 1, 1 July 2002 (2002-07-01), pages 125-138, XP55383405, US ISSN: 1074-7427, DOI: 10.1006/nlme.2001.4047
- GERARD J MAREK ET AL: "The Selective 5-HT2A Receptor Antagonist M100907 Enhances Antidepressant-Like Behavioral Effects of the SSRI Fluoxetine", NEUROPSYCHOPHARMACOLOGY., vol. 30, no. 12, 11 May 2005 (2005-05-11), pages 2205-2215, XP055383408, US ISSN: 0893-133X, DOI: 10.1038/sj.npp.1300762
- J. R. CIRRITO ET AL: "Serotonin signaling is associated with lower amyloid-? levels and plaques in transgenic mice and humans", PROCEEDINGS NATIONAL ACADEMY OF SCIENCES PNAS, vol. 108, no. 36, 22 August 2011 (2011-08-22), pages 14968-14973, XP055383357, US ISSN: 0027-8424, DOI: 10.1073/pnas.1107411108 cited in the application
- JILL S GOLDMAN ET AL: "Genetic counseling and testing for Alzheimer disease: Joint practice guidelines of the American College of Medical Genetics and the National Society of Genetic Counselors", GENETICS IN MEDICINE, vol. 13, no. 6, 16 May 2011 (2011-05-16), pages 597-605, XP55383416, US ISSN: 1098-3600, DOI: 10.1097/GIM.0b013e31821d69b8

## Description

### CROSS REFERENCE TO RELATED APPLICATIONS

The present application claims the benefit of priority of U.S. Provisional Application No. 62/314,857, filed March 29, 2016 and Swedish Application No. 1630067-5, filed April 4, 2016.

Provided herein are compounds, compositions and methods for reducing the rate of accumulation of amyloid plaques and Aβ peptides in subjects using 5-HT2A serotonin receptor inverse agonists or antagonists, or pharmaceutically acceptable salts thereof The present invention relates to a selective 5-HT2A serotonin receptor inverse agonist or antagonist, or a pharmaceutically acceptable salt thereof, for use in a method of delaying the onset and/or preventing Alzheimer's disease (AD) by reducing the concentration of Aβ peptides (Aβ) in a subject, wherein the concentration is in the brain of the subject; said method comprising administering an effective amount of a selective 5-HT2A serotonin receptor inverse agonist or antagonist or a pharmaceutically acceptable salt thereof to the subject in need thereof. The invention also relates to a composition comprising a selective 5-HT2A serotonin receptor inverse agonist or antagonist, or a pharmaceutically acceptable salt thereof, and an effective amount of an agent selected from the group consisting of an antidepressant, an M1 muscarinic acetylcholine receptor agonist, a 5-HT4 serotonin receptor agonist, an anti-amyloid beta monoclonal antibody, and a beta-secretase 1 (BACE1) inhibitor for use in said method.

### BACKGROUND

Amyloid-β (Aβ) dysregulation appears to initiate the pathogenesis of Alzheimer's disease (AD) with a cascade of downstream factors that exacerbate and propagate neuronal injury (St. George-Hyslop and Morris, 2008). Aβ peptides can accumulate as toxic plaques (amyloid plaques) and soluble oligomers in the brains of individuals with AD a decade or more before the symptoms of AD are identified (Morris and Price, 2001). The concentration of Aβ peptides is a critical factor determining if and when it will aggregate into these toxic structures; high concentrations of Aβ are more prone to convert from its normal soluble form into these multimeric conformations (Lomakin et al., 1997). Aβ peptides are formed within neurons by sequential cleavage of the amyloid precursor protein (APP) by two enzymes, β-secretase and then γ-secretase. Alternatively, α-secretase can cleave APP within the Aβ sequence, which precludes the Aβ peptide from being formed at all. The enzymes and mechanisms that produce Aβ peptides have been well characterized; however, the mechanisms that regulate Aβ peptide production and levels are only partly understood. Understanding the cellular processes that regulate Aβ peptide levels may provide greater insight into disease pathogenesis and suggest new avenues to treat or prevent AD.

The imbalance between production and elimination of Aβ peptides and accumulation of amyloid plaque may occur many years prior to an individual being diagnosed with a neurodegenerative disorder such as AD. Such individuals may be identified using Positron Imaging Tomography (PET), a nuclear medicine neuroimaging tool that can be used to make a picture of amyloid plaque accumulation within a person's brain. Still other subjects may have mild cognitive impairment indicating possibly that accumulation of amyloid plaque has started. In addition, person's carrying certain mutations, such as mutations on chromosome 21 that cause the formation of abnormal amyloid precursor protein (APP), or in genes involved in processing APP such as mutations on chromosome 14 that cause abnormal presenilin 1 to be made or mutations on chromosome 1 that lead to abnormal presenilin 2, may benefit from receiving treatments that reduce the rate of accumulation of amyloid plaque before they are diagnosed with AD because each of these mutations affects the breakdown of APP, which in turn leads to accumulation of amyloid plaque. Subjects carrying one or two alleles of the APOE ε4 gene, or persons with a strong family history of AD also may at some point benefit from receiving treatments that reduce the rate of accumulation of amyloid plaque before they are diagnosed with AD.

Other subjects potentially in need of treatments that reduce or prevent the accumulation of amyloid plaque may be identified by changes in cerebrospinal fluid (CSF) biomarkers t-tau and p-tau or CSF amyloid biomarkers Aβ(1-40), Aβ(1-42), and Aβ(1-x) or reductions from baseline in total whole brain, cortical or hippocampal volume, or increases in brain ventricular volume using volumetric magnetic resonance imaging (vMRI).

Another example where Amyloid-β (Aβ) dysregulation leads to disease is Down's syndrome. These subjects are trisomic for the amyloid precursor protein (APP) gene. Therefore, these individuals overproduce Amyloid-β (Aβ), and most subjects with Down's syndrome accumulate substantial amyloid plaques by ages 40 to 50. Thus such individuals would likely benefit from receiving therapies that restore the balance between the production and elimination of Aβ peptides or reduce the rate of accumulation of amyloid plaque at an earlier age, before their plaques are fully formed.

Activation of postsynaptic receptors is known to modulate Aβ levels. For example, muscarinic M1 acetylcholine receptors elevate cleavage of APP by α-secretase, which lowers Aβ production and levels (Eckols et al., 1995; Nitsch et al., 1992). Chronic administration of M1 receptor agonists reduce brain Aβ levels and plaque load (Caccamo et al., 2006; Davis et al., 2010; Nitsch et al., 2000).

Antidepressant medications that elevate synaptic concentrations of serotonin, particularly selective serotonin reuptake inhibitors (SSRIs) such as citalopram and fluoxetine, as well as antidepressants that elevate other neurotransmitters in addition to serotonin such as serotonin norepinephrine reuptake inhibitors (SNRIs) such as desvenlafaxine have been shown to lower beta amyloid levels and Plaque Burden in a Mouse Model of AD and reduce amyloid plaque load in humans (Cirrito et al., 2011). In that same study, Cirrito and co-workers showed that direct infusion of serotonin into the hippocampus reduced ISF Aβ40 and Aβ42 levels. However as serotonin will activate all subtypes of 5HT-receptors, it is unclear from these studies which 5-HT receptor subtype(s) mediate reductions on Aβ.

Several studies have also assessed the effect of serotonin receptors (5HT-Rs) on APP processing and Aβ levels. Superficially, the results of the Cirrito study (Cirrito et al., 2011) would appear to be consistent with other studies (see Shen et al., 2011; Cochet et al., 2013; Tesseur et al., 2013), in that increasing agonist activity at serotonin receptors caused reductions in beta amyloid. Although the above mentioned findings are scientifically interesting they have not generated any benefits to those suffering, or prone to suffer from AD Morley et al. disclose in NEUROBIOLOGY OF LEARNING AND MEMORY, 78(1), 2002, p. 125-138, that pre-treatment with a monoclonal antibody to amyloid beta in 12-month-old senescence accelerated mice (SAMP8) resulted in memory enhancement drugs to facilitate retention. WO-A-2009/039460 discloses in example 3 that mice receiving infusion of amyloid beta protein fragment (25-35)) were pre-treated with pimavanserin. In this Alzheimer's disease model, pimavanserin reversed the augmented locomotor response to amphetamine. EP-A-1576985 discloses compounds having D4 and/or 5-HT2A antagonistic, partial agonistic or inverse agonistic activity in combination with other compounds for treating neurodegenerative diseases. WO-A-2007133802 discloses the use of pimavanserin tartrate for the treatment or prevention of Down's syndrome, Alzheimer's or Parkinson's disease, or Lewy Body dementia. WO-A-0189498 discloses volinanserin for use in treating the behavioral, psychological and motor symptoms of dementia, including Alzheimer's and Parkinson's disease.

### SUMMARY

The present invention relates to a selective 5-HT2A serotonin receptor inverse agonist or antagonist, or a pharmaceutically acceptable salt thereof, for use in a method of delaying the onset and/or preventing Alzheimer's disease (AD) by reducing the concentration of Aβ peptides (Aβ) in a subject, wherein the concentration is in the brain of the subject; said method comprising administering an effective amount of a selective 5-HT2A serotonin receptor inverse agonist or antagonist or a pharmaceutically acceptable salt thereof to the subject in need thereof. The invention also relates to a composition comprising a selective 5-HT2A serotonin receptor inverse agonist or antagonist, or a pharmaceutically acceptable salt thereof, and an effective amount of an agent selected from the group consisting of an antidepressant, an M1 muscarinic acetylcholine receptor agonist, a 5-HT4 serotonin receptor agonist, an anti-amyloid beta monoclonal antibody, and a beta-secretase 1 (BACE1) inhibitor for use in said method. The invention is defined by the claims. Any subject-matter falling outside the scope of the claims is provided for information purposes only. Aspects and embodiments herein relate to a method for reducing the rate of accumulation of amyloid plaques comprising administering an effective amount of a 5-HT2A serotonin receptor inverse agonist or antagonist, or a pharmaceutically acceptable salt thereof to a subject.

Other aspects and embodiments herein relate to a method for reducing the concentration of Aβ peptides (Aβ) in a subject comprising administering an effective amount of a 5-HT2A serotonin receptor inverse agonist or antagonist, or a pharmaceutically acceptable salt thereof, to the subject, wherein the concentration is in the brain of the subject.

Other aspects and embodiments herein relate to a method for controlling the production and/or elimination of Aβ peptides (Aβ) comprising administering an effective amount of a 5-HT2A serotonin receptor inverse agonist or antagonist, or a pharmaceutically acceptable salt thereof to a subject, wherein the production is in the brain of the subject.

Other aspects and embodiments herein relate to identifying the subject prior to being administered the 5-HT2A serotonin receptor inverse agonist or antagonist, wherein the subject is identified by one or more of the following: amyloid plaque imaging using Positron Imaging Tomography (PET); genetic testing for a mutation in the amyloid precursor protein (APP) gene; genetic testing for a gene involved in processing amyloid precursor protein (APP); genetic testing an apolipoprotein E (APOE) ε4 carrier; genetic testing for a strong family history of Alzheimer's disease; genetic testing for trisomy for the amyloid precursor protein (APP) gene; changes in amyloid biomarkers; changes in tau biomarkers; reductions from baseline in total whole brain, cortical or hippocampal volume, or increases in brain ventricular volume; and/or the subject has mild cognitive impairment. In addition, older subjects, subjects with diabetes, high blood pressure, obesity, depression, cognitive inactivity or low education, low physical inactivity, excessive alcohol intake, people who smoke, or people who experience severe or repeated head injuries are at increased risk of developing AD or other forms of dementia, and may be identified prior to being administered the 5-HT2A serotonin receptor inverse agonist or antagonist.

Other aspects and embodiments herein relate to an effective amount of a 5-HT2A serotonin receptor inverse agonist or antagonist, or a pharmaceutically acceptable salt thereof for reducing the rate of accumulation of amyloid plaques in a subject.

Other aspects and embodiments herein relate to an effective amount of a 5-HT2A serotonin receptor inverse agonist or antagonist, or a pharmaceutically acceptable salt thereof for reducing the concentration of Aβ peptides (Aβ) in a subject, wherein the concentration is in the brain of the subject.

Other aspects and embodiments herein relate to an effective amount of a 5-HT2A serotonin receptor inverse agonist or antagonist, or a pharmaceutically acceptable salt thereof for controlling the production and/or elimination of Aβ peptides (Aβ) in a subject, wherein the production is in the brain of the subject.

Other aspects and embodiments herein relate to a composition comprising the 5-HT2A serotonin receptor inverse agonist or antagonist, or a pharmaceutically acceptable salt thereof disclosed herein, for example pimavanserin, and an effective amount of an agent selected from the group consisting of an antidepressant, an M1 muscarinic acetylcholine receptor agonist, an anti-amyloid beta monoclonal antibody, and a beta-secretase 1 (BACE1) inhibitor to the subject.

### DESCRIPTION OF DRAWINGS

Figure 1 discloses data related to on ISF (interstitial fluid) levels of Aβ40 and Aβ42 using MDL 100,907, a selective 5-HT2A Serotonin Receptor Inverse Agonist.
Figure 2 discloses data related to on ISF levels of Aβ40 using pimavanserin.

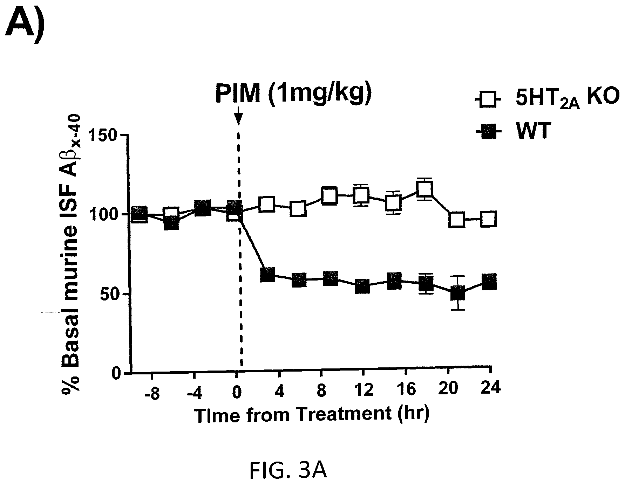

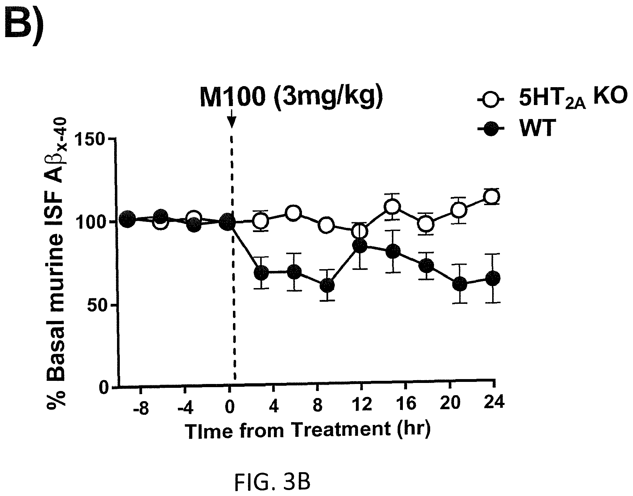

Figure 3 discloses data related to the effects of pimavanserin and MDL 100,907 on ISF levels of Aβ40 in wild-type mice compared to mice in which the 5-HT2A receptor gene has been deleted (5-HT2A KO).

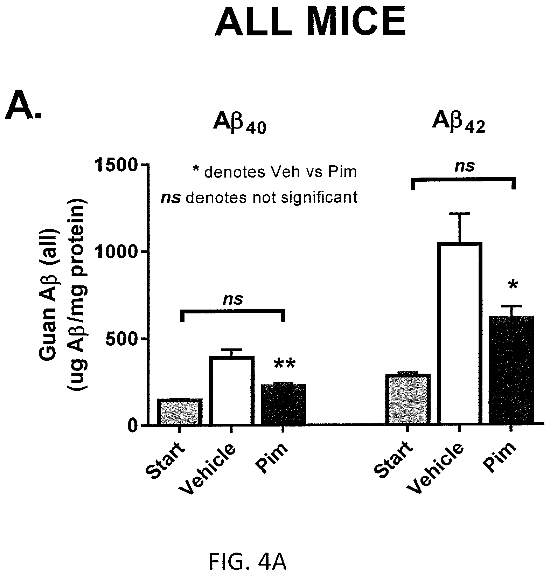

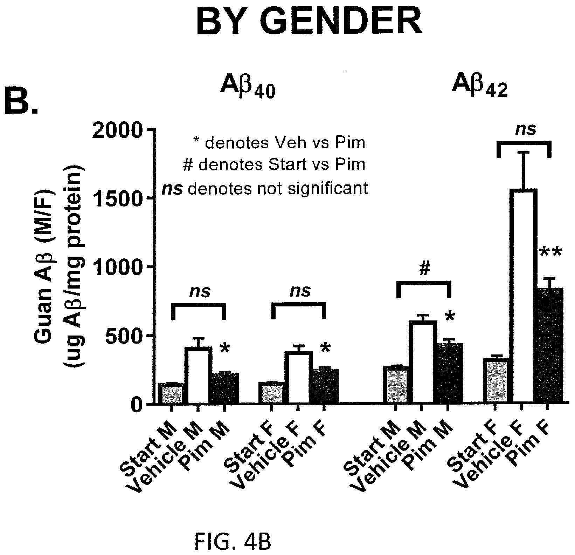

Figure 4 discloses data related to levels of Aβ40 and Aβ42 aggregates in hippocampal extracts after chronic treatment with pimavanserin to mice starting before the mice have developed amyloid plaques.

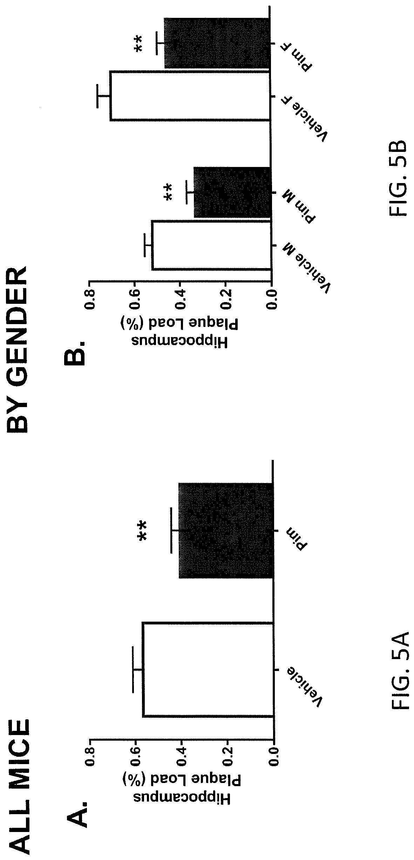

Figure 5 discloses data related to amyloid plaque deposition after chronic treatment with pimavanserin to mice starting before the mice have developed amyloid plaques.

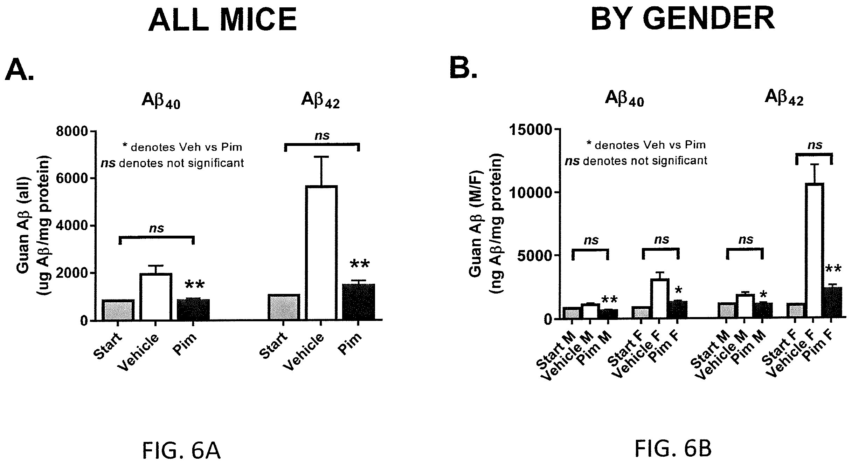

Figure 6 discloses data related to levels of Aβ40 and Aβ42 aggregates in hippocampal extracts after chronic treatment with pimavanserin to mice starting after the mice have started to develop amyloid plaques.

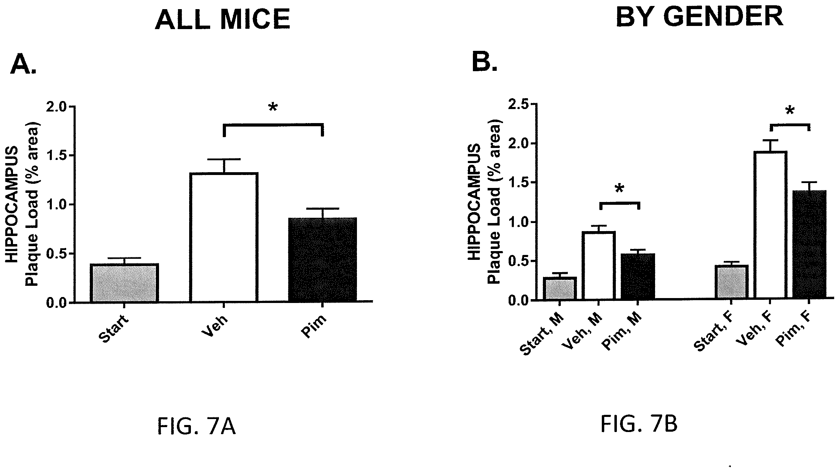

Figure 7 discloses data related to amyloid plaque deposition after chronic treatment with pimavanserin to mice starting after the mice have started to develop amyloid plaques.

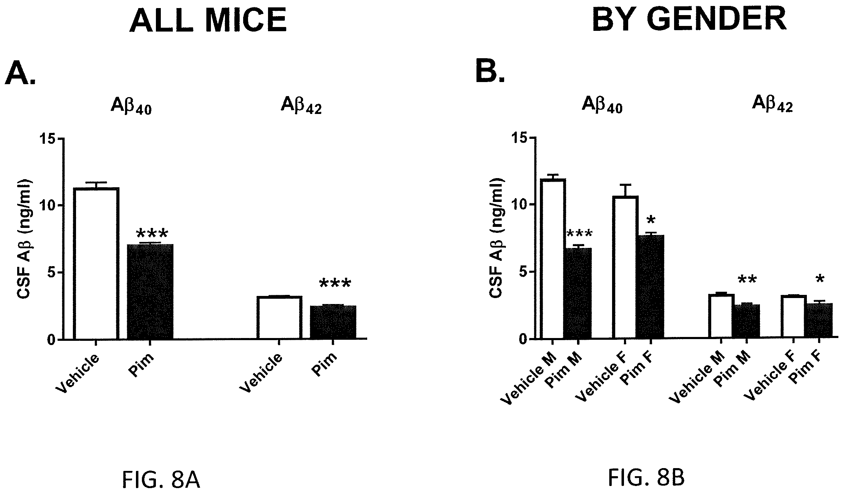

Figure 8 discloses data related to levels of Aβ40 and Aβ42 in CSF after chronic treatment with pimavanserin to mice starting after the mice have started to develop amyloid plaques.

### DETAILED DESCRIPTION

### Definitions:

Unless defined otherwise, all technical and scientific terms used herein have the same meaning as is commonly understood by one of ordinary skill in the art. In the event that there is a plurality of definitions for a term herein, those in this section prevail unless stated otherwise.

The term "neurodegenerative disease or disorder" as used herein refers to a disease or disorder selected from the group consisting of Parkinson's disease (PD), Alzheimer's disease (AD), Down's syndrome, Huntington's disease, frontotemporal lobar degeneration associated with protein TDP-43 (FTLD-TDP), dementia with Lewy bodies (DLB), vascular dementia, amyotrophic lateral sclerosis (ALS), Parkinson's disease with Mild Cognitive Impairment (MCI), Parkinson's disease dementia and other neurodegenerative related dementias due to changes in the brain caused by ageing, disease or trauma; or spinal cord injury.

As used herein, "pharmaceutically acceptable salt" refers to a salt of a compound that does not abrogate the biological activity and properties of the compound. Pharmaceutical salts can be obtained by reaction of a compound disclosed herein with an acid or base. Base-formed salts include, without limitation, ammonium salt (NH₄⁺); alkali metal, such as, without limitation, sodium or potassium, salts; alkaline earth, such as, without limitation, calcium or magnesium, salts; salts of organic bases such as, without limitation, dicyclohexylamine, piperidine, piperazine, methylpiperazine, N-methyl-D-glucamine, diethylamine, ethylenediamine, tris(hydroxymethyl)methylamine; and salts with the amino group of amino acids such as, without limitation, arginine and lysine. Useful acid-based salts include, without limitation, acetates, adipates, aspartates, ascorbates, benzoates, butyrates, caparate, caproate, caprylate, camsylates, citrates, decanoates, formates, fumarates, gluconates, glutarate, glycolates, hexanoates, laurates, lactates, maleates, nitrates, oleates, oxalates, octanoates, propanoates, palmitates, phosphates, sebacates, succinates, stearates, sulfates, sulfonates, such as methanesulfonates, ethanesulfonates, p-toluenesulfonates, salicylates, tartrates, and tosylates.

Pharmaceutically acceptable solvates and hydrates are complexes of a compound with one or more solvent of water molecules, or 1 to about 100, or 1 to about 10, or one to about 2, 3 or 4, solvent or water molecules.

As used herein, a "prodrug" refers to a compound that may not be pharmaceutically active but that is converted into an active drug upon *in vivo* administration. The prodrug may be designed to alter the metabolic stability or the transport characteristics of a drug, to mask side effects or toxicity, to improve the flavor of a drug or to alter other characteristics or properties of a drug. Prodrugs are often useful because they may be easier to administer than the parent drug. They may, for example, be bioavailable by oral administration whereas the parent drug is not. The prodrug may also have better solubility than the active parent drug in pharmaceutical compositions. An example, without limitation, of a prodrug would be a compound disclosed herein, which is administered as an ester (the "prodrug") to facilitate absorption through a cell membrane where water solubility is detrimental to mobility but which then is metabolically hydrolyzed to a carboxylic acid (the active entity) once inside the cell where water-solubility is beneficial. A further example of a prodrug might be a short peptide (polyaminoacid) bonded to an acid group where the peptide is metabolized *in vivo* to release the active parent compound. By virtue of knowledge of pharmacodynamic processes and drug metabolism *in vivo,* those skilled in the art, once a pharmaceutically active compound is known, can design prodrugs of the compound (see, *e.g.* Nogrady (1985) Medicinal Chemistry A Biochemical Approach, Oxford University Press, New York, pages 388-392).

As used herein, to "modulate" the activity of a receptor means either to activate it, i.e., to increase its cellular function over the base level measured in the particular environment in which it is found, or deactivate it, i.e., decrease its cellular function to less than the measured base level in the environment in which it is found and/or render it unable to perform its cellular function at all, even in the presence of a natural binding partner. A natural binding partner is an endogenous molecule that is an agonist for the receptor.

An "agonist" is defined as a compound that increases the basal activity of a receptor (e.g. signal transduction mediated by the receptor).

As used herein, "partial agonist" refers to a compound that has an affinity for a receptor but, unlike an agonist, when bound to the receptor it elicits only a fractional degree of the pharmacological response normally associated with the receptor even if a large number of receptors are occupied by the compound.

An "inverse agonist" is defined as a compound, which reduces, or suppresses the basal activity of a receptor, such that the compound is not technically an antagonist but, rather, is an agonist with negative intrinsic activity.

As used herein, "antagonist" refers to a compound that binds to a receptor to form a complex that does not give rise to any response, as if the receptor was unoccupied. An antagonist attenuates the action of an agonist on a receptor. An antagonist may bind reversibly or irreversibly, effectively eliminating the activity of the receptor permanently or at least until the antagonist is metabolized or dissociates or is otherwise removed by a physical or biological process.

As used herein, a "subject" refers to an animal that is the object of treatment, observation or experiment. "Animal" includes cold- and warm-blooded vertebrates and invertebrates such as birds, fish, shellfish, reptiles and, in particular, mammals. "Mammal" includes, without limitation, mice; rats; rabbits; guinea pigs; dogs; cats; sheep; goats; cows; horses; primates, such as monkeys, chimpanzees, and apes, and, in particular, humans.

As used herein, the term "Aβ" represents amyloid beta, refers collectively to amyloid beta (Aβ) peptide fragments (Aβ peptides) derived from proteolytic processing of the amyloid precursor protein (APP), and includes, but is not limited to, Aβ38, Aβ40, Aβ42 and Aβ43, with the numbers referring to the amino acid length of each peptide fragment. The terms "Aβ", amyloid beta, "Aβ peptides", and amyloid beta peptides are herein used interchangeably.

A subject in need of treatment with an agent to lower levels of Aβ or amyloid plaques could be a subject diagnosed with any of the following conditions: AD, dementia, or Down's syndrome. Preferably the subjects have not yet been diagnosed with any of the foregoing conditions, but nevertheless carry genetic predispositions for developing any of the foregoing conditions, or for overproducing amyloid or Aβ, would also be considered subjects in need of treatment with an agent to lower levels of Aβ or reduce the rate of accumulation of amyloid plaques. Examples of genes in which genetic variation is thought to alter the risk for developing Alzheimer's disease or dementia include, but are not limited to APP (amyloid precursor protein), presenilin 1 or 2 (PSEN1 or PSEN2), and Apolipoprotein E (ApoE) (see Khanahmadi et al., 2015). In addition, subjects who carry an extra copy of certain of these genes, such as the APP gene, such as for example subjects with Down's syndrome, who therefore overproduce amyloid or Aβ would also be considered subjects in need of treatment with an agent to lower levels of Aβ or amyloid. Subjects who have not been diagnosed with any of the foregoing conditions, but who show evidence of amyloid plaque on positron emission tomography (PET) scans, or who show losses of whole brain, cortical or hippocampal volume, or increases in ventricular volume on volumetric magnetic resonance imaging (vMRI) scans may also be considered subjects in need of treatment with an agent to lower levels of Aβ or amyloid plaques. In addition, older subjects, subjects with diabetes, high blood pressure, obesity, depression, cognitive inactivity or low education, low physical inactivity, excessive alcohol intake, people who smoke, or people who experience severe or repeated head injuries are at increased risk of developing AD or other forms of dementia, and may be identified as subjects in need of treatment with an agent to lower levels of Aβ or amyloid plaques The present invention is directed to delaying the onset and/or preventing Alzheimer's disease in a subject in need thereof.

As used herein, amyloid plaque refers to the aggregation or clumping together of Aβ into deposits, such as insoluble deposits, e.g. 'plaques'. These amyloid plaques can be visualized histologically or can be quantified biochemically as the remaining fraction of Aβ that for example can be recovered by extraction using guanidine hydrochloride after the soluble and membrane-bound fractions of Aβ have been recovered by extraction using phosphate buffered saline (PBS) and Triton-X 100, respectively. Although amyloid plaques are mainly located extracellularly, and the guanidine extractable fraction contains both extracellular and intracellular amyloid, they are considered to represent the same thing, namely the amyloid plaque that is pathogenic to a subject. Thus the terms 'aggregated Aβ', guanidine extractable fraction of Aβ, and amyloid plaque shall henceforth be considered equivalent, and used interchangeably herein.

As used herein, the term 'reducing the rate of accumulation of amyloid plaques' shall include reducing, halting, preventing, slowing or reversing the formation of amyloid plaques, the rate of formation of amyloid plaques, the rate of amyloid plaque formation, amyloid plaque formation, the accumulation of amyloid plaques, the rate of accumulation of amyloid plaques, the rate of amyloid plaque accumulation, and/or amyloid plaque accumulation. Additionally reducing rate of expansion of the size and/or number of existing amyloid plaques or reducing the size and/or number of existing amyloid plaques are to be included in the term 'reducing the rate of accumulation of amyloid plaques'. Amyloid plaque load refers to the amount of amyloid plaque in a subject's brain. Amyloid plaque load can be quantified immunohistochemically as the percent area of the brain that stains positive for amyloid plaque using an anti-Aβ antibody. Reducing amyloid plaque load, reducing amyloid plaque growth, reducing accumulation of amyloid plaque, and reducing the rate of accumulation of amyloid plaques shall henceforth be considered equivalent, and used interchangeably herein. Aggregation of Aβ refers to the formation of oligomers of Aβ peptides which cluster together to form fibrils, which in turn adhere together to form mats, which clump together to finally form plaques. Aggregated Aβ therefore may refer to any or all of oligomers of Aβ, Aβ fibrils, mats of Aβ, or Aβ plaques.

As used herein, a "patient" refers to a subject that is being treated by a medical professional such as, e.g. an M.D. or a D.V.M., to attempt to cure, or at least ameliorate the effects of, a particular disease or disorder or to prevent the disease or disorder from occurring in the first place.

As used herein, a "carrier" refers to a compound that facilitates the incorporation of a compound into cells or tissues. For example, without limitation, dimethyl sulfoxide (DMSO) is a commonly utilized carrier that facilitates the uptake of many organic compounds into cells or tissues of a subject.

As used herein, a "diluent" refers to an ingredient in a pharmaceutical composition that lacks pharmacological activity but may be pharmaceutically necessary or desirable. For example, a diluent may be used to increase the bulk of a potent drug whose mass is too small for manufacture or administration. It may also be a liquid for the dissolution of a drug to be administered by injection, ingestion or inhalation. A common form of diluent in the art is a buffered aqueous solution such as, without limitation, phosphate buffered saline that mimics the composition of human blood.

As used herein, an "excipient" refers to an inert substance that is added to a pharmaceutical composition to provide, without limitation, bulk, consistency, stability, binding ability, lubrication, disintegrating ability etc., to the composition. A "diluent" is a type of excipient.

A "receptor" is intended to include any molecule present inside or on the surface of a cell that may affect cellular physiology when it is inhibited or stimulated by a ligand. Typically, a receptor comprises an extracellular domain with ligand-binding properties, a transmembrane domain that anchors the receptor in the cell membrane, and a cytoplasmic domain that generates a cellular signal in response to ligand binding ("signal transduction"). A receptor also includes any intracellular molecule that in response to ligation generates a signal. A receptor also includes any molecule having the characteristic structure of a receptor, but with no identifiable ligand. In addition, a receptor includes a truncated, modified, mutated receptor, or any molecule comprising partial or all of the sequences of a receptor.

A "5-HT2A serotonin receptor" relates to a distinct genetic subtype of the 5-HT₂ serotonin receptor sub-family that belongs to the serotonin receptor family that is a member of the G-protein-coupled receptor(GPCR) superfamily.

A "G-protein", is a protein that binds and hydrolyzes gaunosine triphosphate (GTP) and links ligand activation of cell-membrane receptors to intracellular signal transduction.

"Ligand" is intended to include any substance that interacts with a receptor.

"Selective" or "selectivity" is defined as a compound's ability to generate a desired response from a particular receptor type, subtype, class or subclass while generating less or little or no response from other receptor types. In the case of an "agonist" or "partial agonist", "selectivity" means the ability of a compound to increase the basal activity of a receptor (e.g. signal transduction mediated by the receptor) from a particular receptor type, subtype, class or subclass while generating less or little or no response from other receptor types. In the case of an "inverse agonist", "selectivity" means the ability of a compound to reduce, or suppress the basal activity of a receptorfrom a particular receptor type, subtype, class or subclass while generating less or little or no response from other receptor types. In the case of an "antagonist", "selectivity" means the ability of a compound to attenuate the action of an agonist of a receptor from a particular receptor type, subtype, class or subclass, while not attenuating the actions of agonists on other receptor types.

Selectivity can be quantified by measuring the concentration of a compound required to generate a desired response from a particular receptor subtype while generating less or little or no response from another receptor subtype. For an agonist or partial agonist, the desired response would be to increase the basal activity of a particular receptor subtype. For an inverse agonist, the desired response would be to reduce, or suppress the basal activity of a particular receptor subtype. A compound can be deemed 'selective' for a particular receptor subtype if the concentration of said compound required to generate a desired response from a particular receptor subtype is for example 10-fold lower, or 100-fold lower or 1000-fold lower than would be required to generate a response from other receptor subtypes. For example, an inverse agonist that is selective for 5-HT2A receptors over D2 dopamine receptors would be capable of suppressing the basal activity of 5-HT2A receptors at concentrations at least 10 or 100 or 1000-fold lower than would be required to suppress the basal activity of D2 receptors. For example, an antagonist that is selective for the 5-HT2A receptors over D2 dopamine receptors would be capable of blocking agonist activation of 5-HT2A receptors at concentrations at least 10 or 100 or 1000-fold lower than would be required to block agonist activation of D2 receptors. For example, an antagonist that is selective for 5-HT2A receptors over the serotonin transporter would be capable of blocking agonist activation of 5-HT2A receptors at concentrations at least 10 or 100 or 1000-fold lower than would be required to block reuptake of serotonin through serotonin transporters.

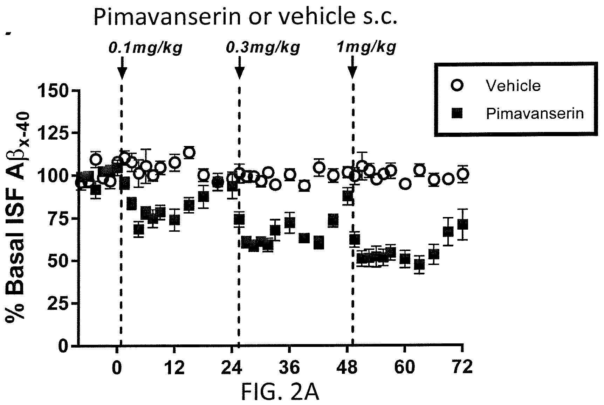

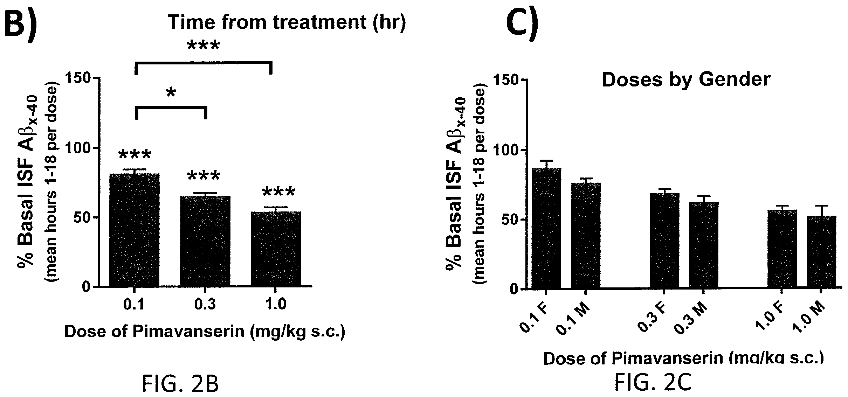

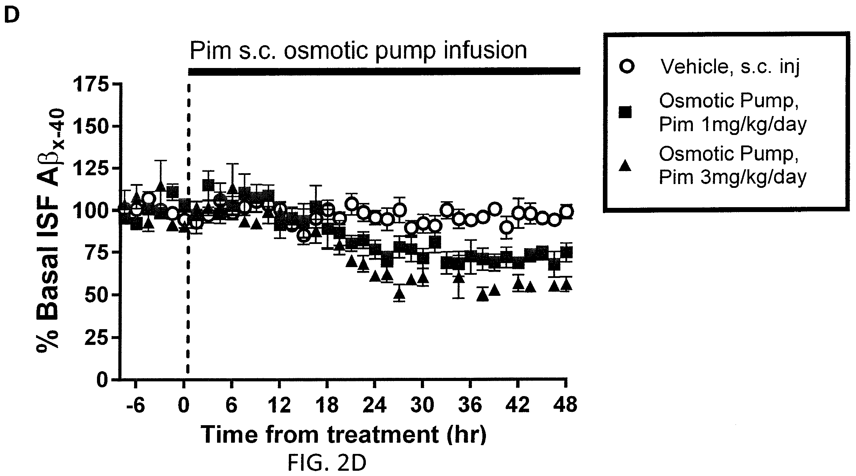

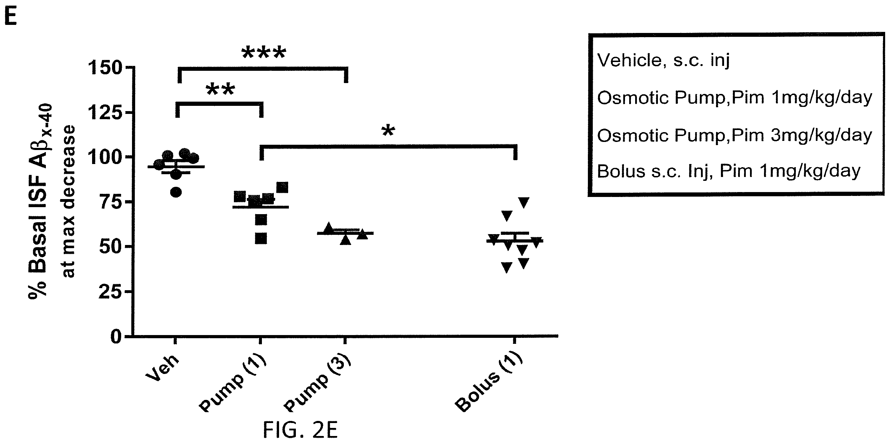

Receptor Selection and Amplification Technology (R-SAT™) is a particularly useful means to assess basal activity of GPCRs (Burstein et al., 2006). Hence, determining the potencies of compounds at either increasing or decreasing the basal activity of receptor subtypes of interest using R-SAT™ is a particularly useful means to assess the receptor selectivity of agonists, partial agonists and inverse agonists. Hence R-SAT™ may be used in order to classify compounds, for example as selective 5-HT2A inverse agonists. An example is shown in Figure 2 in Hacksell et al., 2014.

As used herein, "coadministration" of pharmacologically active compounds refers to the delivery of two or more separate chemical entities, whether in vitro or in vivo. Coadministration means the simultaneous delivery of separate agents; the simultaneous delivery of a mixture of agents; as well as the delivery of one agent followed by delivery of a second agent or additional agents. Agents that are coadministered are typically intended to work in conjunction with each other.

The term "an effective amount" as used herein means an amount of active compound or pharmaceutical agent that elicits the biological or medicinal response in a tissue, system, animal or human that is being sought by a researcher, veterinarian, medical doctor or other clinician, which includes alleviation or palliation of the symptoms of the disease being treated.

When used herein, "prevent/preventing" should not be construed to mean that a condition and/or a disease never might occur again after use of a compound or pharmaceutical composition according to embodiments disclosed herein to achieve prevention. Further, the term should neither be construed to mean that a condition not might occur, at least to some extent, after such use to prevent said condition. Rather, "prevent/preventing" is intended to mean that the condition to be prevented, if occurring despite such use, may be less severe than without such use.

The term "ip" denotes an intraperitoneal injection.

The term "s.c." , "s.c. inj" denotes a subcutaneous injection.

The letters "M" and "F" used in the figures denote male and female respectively.

The term "WT" denotes wild-type, e.g. wild-type mice.

The term "veh" denotes vehicle.

Embodiments disclosed herein relate to compounds, compositions and methods for reducing the rate of accumulation of amyloid plaques comprising administering an effective amount of a 5-HT2A serotonin receptor inverse agonist or antagonist, or a pharmaceutically acceptable salt thereof to a subject, such as a subject in need. A subject in need is for example a person shows signs of accumulation of amyloid plaques in the brain, e.g. in the central nervous system. Examples of ways to identify a subject in need, and/or a subject shows signs of accumulation of amyloid plaques are: amyloid plaque imaging by Positron Imaging Tomography (PET); genetic testing for a mutation in the amyloid precursor protein (APP) gene; genetic testing for a gene involved in processing amyloid precursor protein (APP); genetic testing for an apolipoprotein E (APOE) ε4 carrier; genetic testing for a strong family history of Alzheimer's disease; genetic testing for trisomy of the amyloid precursor protein (APP) gene; identifying changes in amyloid biomarkers; identifying changes in tau biomarkers; and identifying decreases from baseline in total brain, cortical or hippocampal volume, or increases from baseline in ventricular volume for example using volumetric magnetic resonance imaging (vMRI). Additionally a subject having mild cognitive impairment, thus being at risk of developing a neurodegenerative disease such as Alzheimer's disease may be considered a subject in need of reducing the rate of accumulation of amyloid plaques in the brain e.g. in the central nervous system. In addition, older subjects, subjects with diabetes, high blood pressure, obesity, depression, cognitive inactivity or low education, low physical inactivity, excessive alcohol intake, people who smoke, or people who experience severe or repeated head injuries are at increased risk of developing AD or other forms of dementia, and may be identified as subjects in need of treatment with an agent to lower levels of Aβ or amyloid plaques The invention is directed to delaying the onset and/or preventing Alzheimer's disease in a subject in need thereof.

Further embodiments disclosed herein relate to compounds, compositions and methods for controlling the production and/or elimination of amyloid-beta (Aβ), reducing the concentration of amyloid-beta (Aβ) and/or reducing the rate of accumulation of aggregated Aβ or amyloid plaques by administering an effective amount of a 5-HT2A serotonin receptor inverse agonist, or a pharmaceutically acceptable salt thereof to a subject.

Further embodiments disclosed herein relate to compounds, compositions and methods for restoring the balance between production and elimination of amyloid-beta (Aβ), controlling the concentration of amyloid-beta (Aβ) or controlling the rate of accumulation of aggregated Aβ or amyloid plaques by administering an effective amount of a 5-HT2A serotonin receptor inverse agonist, or a pharmaceutically acceptable salt thereof to a subject.

Further embodiments disclosed herein relate to controlling the production and/or elimination, for example by decreasing the production and/or increasing the elimination of Aβ peptides, in a subject by administering a 5-HT2A serotonin receptor inverse agonist or antagonist.

In some embodiments the Aβ is Aβ40 and Aβ42.

In some embodiments the Aβ is Aβ42.

In some embodiments the Aβ, such as Aβ40 and/or Aβ42, can be detected in cerebrospinal fluid (CSF) of the subject.

The imbalance between production and elimination of Aβ peptides and accumulation of amyloid plaque may occur many years prior to an individual being diagnosed with a neurodegenerative disorder such as AD. Such individuals may be identified using Positron Imaging Tomography (PET), a nuclear medicine neuroimaging tool that can be used to make a picture of amyloid plaque accumulation within a person's brain. Still other subjects may have mild cognitive impairment indicating possibly that accumulation of amyloid plaque has started. In addition, person's carrying certain mutations, such as mutations on chromosome 21 that cause the formation of abnormal amyloid precursor protein (APP), or in genes involved in processing APP such as mutations on chromosome 14 that cause abnormal presenilin 1 to be made or mutations on chromosome 1 that lead to abnormal presenilin 2, may benefit from receiving treatments that reduce the rate of accumulation of amyloid plaque before they are diagnosed with AD because each of these mutations affects the breakdown of APP, which in turn leads to accumulation of amyloid plaque. Subjects carrying one or two alleles of the APOE ε4 gene, or persons with a strong family history of AD also may at some point benefit from receiving treatments that reduce the rate of accumulation of amyloid plaque before they are diagnosed with AD. In addition, older subjects, subjects with diabetes, high blood pressure, obesity, depression, cognitive inactivity or low education, low physical inactivity, excessive alcohol intake, people who smoke, or people who experience severe or repeated head injuries are at increased risk of developing AD or other forms of dementia, and may be identified as subjects that would at some point benefit from receiving treatments that reduce the rate of accumulation of amyloid plaque before they are diagnosed with AD. The above mentioned identifications may be used in combination, for example, a person carrying any of the mentioned mutations could subsequently to identifying the mutation be investigated by PET.

Other subjects potentially in need of treatments that reduce or prevent the accumulation of amyloid plaque may be identified by changes in cerebrospinal fluid (CSF) biomarkers t-tau and p-tau or CSF amyloid biomarkers Aβ(1-40), Aβ(1-42), and Aβ(1-x) or reductions from baseline in total brain, cortical, or hippocampal volume, or increases from baseline in ventricular volume using volumetric magnetic resonance imaging (vMRI).

Another example where Amyloid-β (Aβ) dysregulation leads to disease is Down's syndrome. These subjects are trisomic for the amyloid precursor protein (APP) gene. Therefore, these individuals overproduce Amyloid-β (Aβ), and most subjects with Down's syndrome accumulate substantial amyloid plaques by ages 40 to 50. Thus such individuals would likely benefit from receiving therapies that restore the balance between the production and elimination of Aβ peptides and/or reduce the rate of accumulation of amyloid plaque at an earlier age, before their plaques are fully formed.

In view of the findings of Cirrito and others disclosed herein it was surprising that inverse agonists or antagonists of the 5-HT2A receptor reduce concentrations of Aβ, and reduce the rate of accumulation of amyloid plaques.

Provided herein are 5-HT2A serotonin receptor inverse agonists (compounds with pharmacological actions mediated primarily through inverse agonism at 5-HT2A receptors) or antagonists for restoring the balance between production and elimination of Aβ and/or reducing the rate of accumulation of amyloid plaques or aggregated Aβ. Selective 5-HT2A serotonin receptor inverse agonists are provided in some embodiments disclosed herein.

Examples of 5-HT2A inverse agonists are pimavanserin (Nuplazid™, ACP-103), volinanserin (MDL 100,907), eplivanserin (SR-46349B), ritanserin, ketanserin, cianserin, fananserin, pruvanserin (EMD-281,014, LY-2,422,347), glemanserin (MDL-11,939), nelotanserin (APD125), AVE8488, ITI-007 (1-(4-Fluorophenyl)-4-(3-methyl-2,3,6b,9,10,10a-hexahydro-1H-pyrido[3',4':4,5]pyrrolo[1,2,3-de]quin-oxalin-8(7H)-yl)-1-butanone), and Temanogrel. In an embodiment the 5-HT2A inverse agonist is a selective 5-HT2A inverse agonist. In an embodiment the selective 5-HT2A inverse agonist is selected from the group consisting of pimavanserin, volinanserin, eplivanserin, nelotanserin, glemanserin, pruvanserin, ITI-007 and temanogrel. In an embodiment the selective 5-HT2A inverse agonist is selected from pimavanserin or volinanserin. In an embodiment the selective 5-HT2A inverse agonist is pimavanserin.

Pimavanserin (or PIM), which is also known as N-(1-methylpiperidin-4-yl)-N-(4-fluorophenylmethyl)-N'-(4-(2-methylpropyloxy)phenylmethyl)carbamide, N-[(4-fluorophenyl)methyl]-N-(l-methyl-4-piperidinyl)-N'-[[4-(2-methylpropoxy)phenyl]methyl]-urea, 1-(4-fluorobenzyl)-1-(1-methylpiperidin-4-yl)-3-[4-(2-methylpropoxy)benzyl]urea, or ACP-103. Pimavanserin commonly is administered as pimavanserin tartrate and has the structure of Formula (I):

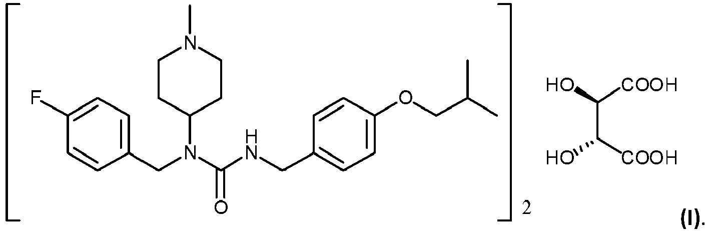

Pimavanserin may be synthesized by methods described in U.S. Patent No. 7,601,740 (see columns 22-26). In a specific embodiment, pimavanserin is prepared as shown in Scheme I below, or by modification of these methods. Ways of modifying the methodology include, among others, modification in temperature, solvent, reagents, etc., as will be known those skilled in the art.

Pimavanserin can be present in a number of salts and crystalline forms which are included in the present disclosure.

Exemplary salts include the tartrate, hemi-tartrate, citrate, fumarate, maleate, malate, phosphate, succinate, sulphate, and edisylate (ethanedisulfonate) salts. Pimavanserin salts including the aforementioned ions, among others, are described in U.S. Patent No. 7,868,176.

Disclosed herein is also MDL 100,907 (or M100), also known as volinanserin or (*R*)-(+)-α-(2,3-Dimethoxyphenyl)-1-[2-(4-fluorophenyl)ethyl]-4-piperinemethanol.

As discussed herein it would be advantageous to reduce concentrations of Aβ peptides, to prevent or reduce the rate of formation of amyloid plaques, to reduce the expansion of existing amyloid plaques, and/or to reduce the size and/or number of existing amyloid plaques. Plaque size in humans can be assessed using positron emission tomography (PET) imaging with agents such as Pittsburg Compound B (PIB) that specifically label amyloid plaque in the brain (an example of how such an assessment can be made is disclosed by Cirrito et al., 2011; supplemental information, page 2). Concentrations of Aβ in brain tissue can be assessed as described (see Cirrito et al., 2011; supplemental information, page 2). Concentrations of soluble Aβ in ISF can be assessed as described (see Cirrito et al., 2011; supplemental information, page 1).

Embodiments disclosed herein relate to compounds, compositions and methods for reducing the rate of accumulation of amyloid plaques by administering an effective amount of a selective 5-HT2A inverse agonist to a subject in need of such treatment.

Additionally embodiments disclosed herein relate to compounds, compositions and methods for reducing the concentrations of Aβ peptides, such as Aβ40 and Aβ42, for example detectable in cerebrospinal fluid (CSF), by administering an effective amount of a selective 5-HT2A inverse agonist to a subject. This can be assessed using microelectrodes that specifically sense and quantify soluble Aβ (in vivo Aβ microdialysis) in the interstitial fluid (ISF) (Cirrito et al., 2011, supplemental information page 1) or by the stable isotope labeling kinetic Aβ assay on CSF samples (Sheline et al., 2014, page 10) or by an enzyme-linked immunosorbent assay for Aβ (ELISA-Aβ) as previously described (Cirrito et al., 2011, supplemental information, page 1; Sheline et al., 2014, page 8) or by western blot (Kamenetz et al., 2003). Optionally Aβ peptides may be assessed by measuring their levels in plasma as described by Schupf et al., 2007.

Additionally, embodiments disclosed herein relate to compounds, compositions and methods for reducing the rate of accumulation of aggregated Aβ peptides, such as Aβ40, and/or Aβ42 by administering an effective amount of a selective 5-HT2A inverse agonist to a subject. This can be assessed using an enzyme-linked immunosorbent assay for Aβ (ELISA-Aβ) as described by Kamenetz et al., 2003.

Provided herein are also compounds, compositions and methods for reducing the levels of Aβ peptides in the interstitial fluid surrounding the brain by administering an effective amount of a selective 5-HT2A inverse agonist to a subject.

Provided herein are also compounds, compositions and methods for reducing the levels of Aβ peptides in the interstitial fluid (surrounding the brain) by administering an effective amount of a selective 5-HT2A inverse agonist to a subject in need of such treatment who is not suffering from a neurodegenerative disease but is at risk of developing a neurodegenerative disease.

Provided herein are also compounds, compositions and methods for reducing the levels of Aβ peptides in the CSF of a subject by administering an effective amount of a selective 5-HT2A inverse agonist to a subject in need of such treatment who is not suffering from a neurodegenerative disease but is at risk of developing a neurodegenerative disease.

Provided herein are also compounds, compositions and methods for reducing the levels of Aβ peptides in the CSF of a subject. Provided herein are also methods for reducing the levels of Aβ peptides in the brain of a subject by administering an effective amount of a selective 5-HT2A inverse agonist to the subject. Said subject should be in need of such treatment but not necessarily suffering from a neurodegenerative disease but is at risk of developing a neurodegenerative disease, such as Alzheimer's disease, or Down's syndrome.

Provided herein are also compounds, compositions and methods for reducing the levels of Aβ peptides in the brain of a subject suffering from or at risk of developing a neurodegenerative disease to slow down the emergence or progression of the disease.

Provided herein are also compounds, compositions and methods for reducing the levels of aggregated Aβ peptides in the brain of a subject by administering an effective amount of a selective 5-HT2A inverse agonist to a subject in need of such treatment who is not suffering from a neurodegenerative disease but is at risk of developing a neurodegenerative disease.

Provided herein are also compounds, compositions and methods for reducing the levels of aggregated Aβ peptides in the brain of a subject suffering from or at risk of developing a neurodegenerative disease, e.g. to slow down the emergence or progression of the disease.

Provided herein are compounds, compositions and methods for reducing the rate of accumulation of amyloid plaques in the brain of a subject by administering an effective amount of a selective 5-HT2A inverse agonist to a subject in need of such treatment who is not suffering from a neurodegenerative disease but is at risk of developing a neurodegenerative disease, or to a subject having a neurodegenerative disease, e.g. to slow down the emergence or progression of the disease.

Provided are also compounds, compositions and methods for reducing the rate of accumulation of amyloid plaques. Said methods include administering a 5-HT2A inverse agonist or antagonist to a subject, such as a selective 5-HT2A inverse agonist, such as pimavanserin. Such methods would be useful to subjects who are not suffering from a neurodegenerative disease but at risk of developing a neurodegenerative disease as well as to subjects suffering from a neurodegenerative disease, e.g. to slow down the emergence or progression of the disease.

Provided are compounds, compositions and methods for preventing the expansion, or reducing rate of expansion of the size and/or number of existing amyloid plaques in a subject. As persons skilled in the art will know, the amount of amyloid plaque in the brain may be assessed non-invasively using positron emission tomography (PET) imaging with agents such as Pittsburg Compound B (PIB) that specifically label amyloid plaque in the brain (see Cirrito et al., 2011, supplemental information page 2). Said methods include administering a selective 5-HT2A inverse agonist to a subject, for example to a subject suffering from a neurodegenerative disease.

Provided are compounds, compositions and methods for reducing the size and/or number of existing amyloid plaques. Said methods include administering a 5-HT2A inverse agonist or antagonists (e.g. a selective 5-HT2A inverse agonist) to a subject.

Provided are compounds, compositions and methods for reducing the rate of accumulation of amyloid plaques in a subject, wherein the subject is at an early stage of a neurodegenerative disease or the subject is at risk of developing a neurodegenerative disease (i.e. a subject in need). Said methods include administering a selective 5-HT2A inverse agonist or antagonist (e.g. a selective 5-HT2A inverse agonist, such as pimavanserin) to the subject, who is at risk of developing a neurodegenerative disease. Some of the methods disclosed herein above are provided by administering a compound with its principle activity as a 5-HT2A inverse agonist, e.g. a selective 5-HT2A inverse agonist. Selectivity can be quantified by measuring the concentration of a compound required to generate a desired response from a particular receptor subtype while generating less or little or no response from another receptor subtype. For example, an inverse agonist that is selective for 5-HT2A receptors over D2 dopamine receptors would be capable of suppressing the basal activity of 5-HT2A receptors at concentrations at least 10 or 100 or 1000-fold lower than would be required to suppress the basal activity of D2 receptors. The methods disclosed herein include administering an effective amount of a selective 5-HT_{2A} inverse agonist to the subject. According to one aspect the 5-HT2A inverse agonist is administered in an amount sufficient to interact and/or bind to the 5-HT2A receptor. According to one aspect the 5-HT2A inverse agonist is Volinanserin (MDL 100,907), Eplivanserin (SR-46349B), Ritanserin, Ketanserin, Cianserin, Fananserin, Pruvanserin (EMD-281,014, LY-2,422,347), pimavanserin (Nuplazid™, ACP-103), Glemanserin (MDL-11,939), Nelotanserin (APD125), AVE8488, ITI-007 (1-(4-Fluorophenyl)-4-(3-methyl-2,3,6b,9,10,10a-hexahydro-1H-pyrido[3',4':4,5]pyrrolo[1,2,3-de]quinoxalin-8(7H)-yl)-1-butanone), or Temanogrel. In some aspects the 5-HT2A inverse agonist is a selective 5-HT2A antagonist such as volinanserin (MDL 100,907), eplivanserin (SR-46349B), pruvanserin, pimavanserin (ACP-103, Nuplazid™), glemanserin, nelotanserin (APD125), ITI-007 and Temanogrel. In some aspects the selective 5-HT2A inverse agonist is pimavanserin or volinanserin. In some aspects the selective 5-HT2A inverse agonist is pimavanserin.

According to one aspect disclosed herein the selective 5-HT2A inverse agonist is administered to a subject suffering (for example at an early stage of the disease, or who has not yet developed substantial symptoms of the disease, or to slow down or prevent the emergence or progression of the disease), or at risk of developing a neurodegenerative disease as defined herein. In one aspect the neurodegenerative disease is Alzheimer's disease (AD) or Parkinson's disease (PD), Down's syndrome or Lewy body disease (LBD).

According to one aspect disclosed herein the 5-HT2A inverse agonist is selected from the group consisting of Volinanserin (MDL 100,907), Eplivanserin (SR-46349B), Ritanserin, Ketanserin, Cianserin, Fananserin, Pruvanserin (EMD-281,014, LY-2,422,347), pimavanserin (Nuplazid™, ACP-103), Glemanserin (MDL-11,939), Nelotanserin (APD125), AVE8488, ITI-007 (1-(4-Fluorophenyl)-4-(3-methyl-2,3,6b,9,10,10a-hexahydro-1H-pyrido[3',4':4,5]pyrrolo[1,2,3-de]quinoxalin-8(7H)-yl)-1-butanone) or Temanogrel. In some aspects the 5-HT2A inverse agonist is pimavanserin.

According to one aspect disclosed herein the methods are provided by administering one or more compounds, or pharmaceutically acceptable salts thereof, selected from the group consisting of Volinanserin (MDL 100,907), Eplivanserin (SR-46349B), Ketanserin, Cianserin, Fananserin, Pruvanserin (EMD-281,014, LY-2,422,347), pimavanserin (Nuplazid™, ACP-103), Glemanserin (MDL-11,939), Nelotanserin (APD125), AVE8488, ITI-007 and Temanogrel to a subject at risk of developing or having a neurodegenerative disease, e.g. to slow down the emergence or progression of the disease. Examples of diseases are Alzheimer's disease (AD) or Parkinson's disease (PD), Down's syndrome or Lewy body disease (LBD) or to a subject at risk of developing these disorders. In some embodiments the neurodegenerative disease is Alzheimer's disease or Down's syndrome.

According to one aspect disclosed herein a 5-HT2A inverse agonist such as Volinanserin (MDL 100,907), Eplivanserin (SR-46349B), Ritanserin, Ketanserin, Cianserin, Fananserin, Pruvanserin (EMD-281,014, LY-2,422,347), pimavanserin (Nuplazid™, ACP-103), Glemanserin (MDL-11,939), Nelotanserin (APD125), AVE8488, ITI-007 or Temanogrel is administered to a subject forming amyloid plaque in the brain of said subject, and therefore at risk of developing a neurodegenerative disease, such as Alzheimer's disease (AD) or Parkinson's disease (PD), or Down's syndrome or Lewy body disease (LBD) and said 5-HT2A inverse agonist halts or reduces the rate of formation of the amyloid plaques, halts or reduces the rate of expansion of the size and/or number of existing amyloid plaques, and/or reduces the size and/or number of existing amyloid plaques.

It is appreciated that some antidepressant medications, such as mirtazapine (Avanza, Axit, Mirtaz, Mirtazon, Remeron, Zispin), amoxapine, loxapine, mianserin, trazodone, etoperidone, Etoperidone (Axiomin, Etonin), Lorpiprazole (Normarex), Lubazodone (YM-992, YM-35995 - discontinued), Mepiprazole (Psigodal), Nefazodone (Serzone, Nefadar), amitryptiline, butryptiline, nortryptiline, clomipramine, desipramine, doxepin, imipramine, iprindole, lofepramine, protryptiline, trimipramine, dosulepin, and amitriptylinoxide also have affinity for 5-HT2A receptors, and thus may act as antagonists and/or inverse agonists at 5-HT2A receptors, and therefore may also reduce brain beta amyloid levels through inverse agonism at 5-HT2A receptors in addition to their principle actions as serotonin and norepinephrine reuptake inhibitors. This is different than selective 5-HT2A inverse agonists whose principle actions are derived from their activities as 5-HT2A inverse agonists.

It is appreciated that some antipsychotic medications such as clozapine, N-desmethyl clozapine, risperidone, 9-OH risperidone (paliperidone), quetiapine, olanzapine, pipamperone, brexpiprazole, aripiprazole, asenapine, lurasidone, sertindole, octoclothepin, telfudazine, spiperone, tiospirone, pimozide, clothiapine, cis-flupentixol, fluspiriline, butaclamol, chlorpromazine, amperozide, fluphenazine, chlorproethazine, trifluperidol, perlapine, promazine, moperone, thioridazine, mesorid-azine, melperone, trifluoperazine, trans-flupentixol, bromperidol, prothipendyl, zotepine, blonanserin, iloperidone, perospirone, cariprazine, aripiprazole, RP5063, and ziprasidone have affinity for 5-HT2A receptors and may be either 5-HT2A antagonists and/or 5-HT2A inverse agonists. It is also appreciated that these drugs interact with many receptors in addition to 5-HT2A receptors, such D2 dopamine receptors, and therefore their pharmacological actions are thought to derive from interactions with many receptors in addition to 5-HT2A receptors. This is different than selective 5-HT2A inverse agonists whose principle actions are derived from their activities as 5-HT2A inverse agonists.

Some embodiments disclosed herein are thus 5-HT2A inverse agonists, and generally are thought to exert their pharmacological actions primarily through 5-HT2A receptors. Examples are volinanserin (MDL 100,907, (R)-(2,3-dimethoxyphenyl)-[1-[2-(4-fluorophenyl)ethyl]-4-piperidyl]-methanol), eplivanserin (SR-46349B, (Z,E)-1-(2-fluorophenyl)-3-(4-hydroxyphenyl)-2-propen-1-one O-[2-(dimethylamino)ethyl]oxime), ritanserin (6-[2-[4-[bis(4-fluorophenyl)methylidene]piperidin-1-yl]ethyl]-7-methyl-[1,3]thiazolo[2,3-b]pyrimidin-5-one), ketanserin (3-{2-[4-(4-fluorobenzoyl)piperidin-1-yl]ethyl}quinazoline-2,4(1H,3H)-dione), cianserin, fananserin (2-(3-(4-(p-Fluorophenyl)-1-piperazinyl)-propyl)-2H-naphth(1,8-cd)isothiazole 1,1-dioxide), pruvanserin (EMD-281,014, LY-2,422,347, 7-({4-[2-(4-fluorophenyl)ethyl]piperazin-1-yl}carbonyl)-1H-indole-3-carbonitrile), pimavanserin, (ACP-103, N-(4-fluorophenylmethyl)-N-(1-methylpiperidin-4-yl)-N'-(4-(2-methylpropyloxy)phenylmethyl)carbamide), glemanserin (MDL-11,939, α-phenyl-1-(2-phenylethyl)-4-piperidine methanol), nelotanserin (APD125, 1-[3-(4-bromo-2-methyl-2H-pyrazol-3-yl)-4-methoxyphenyl]-3-(2,4-difluorophenyl)urea), AVE8488, ITI-007 (1-(4-Fluorophenyl)-4-(3-methyl-2,3,6b,9,10,10a-hexahydro-1H-pyrido[3',4':4,5]pyrrolo[1,2,3-de]quinoxalin-8(7H)-yl)-1-butanone) and Temanogrel.

The compound to be used in the methods and/or compositions disclosed herein belong to the group of 5-HT2A serotonin receptor inverse agonist or antagonists. Some examples of selective 5-HT2A serotonin receptor inverse agonist are volinanserin (MDL 100,907), eplivanserin (SR-46349B, for example oral doses at about 1, 2, 5 or 10 mg/day), pruvanserin, pimavanserin (ACP-103, Nuplazid™), glemanserin, nelotanserin (APD125, for example oral doses of about 10, 20, 40 or 80 mg/day), ITI-007 (for example at oral doses of about 10, 20, 40, 60 or 120 mg/day) and Temanogrel. In some embodiments the selective 5-HT2A serotonin receptor inverse agonist is pimavanserin (ACP-103, Nuplazid™).

In some embodiments the selective 5-HT2A serotonin receptor inverse agonist is pimavanserin (ACP-103, Nuplazid™). Pimavanserin can be administered in several ways, for example in oral doses once daily such as 2 to 80 mg/day, such as about 8.5 mg/day, about 10 mg/day, about 17 mg/day, about 20mg/day, about 34 mg/day, about 40 mg/day, about 51 mg/day or about 60 mg/day.

In some embodiments the selective 5-HT2A serotonin receptor inverse agonist is Volinanserin (MDL 100,907). Volinanserin can for example be administered in several ways, for example in oral doses such as 1 to 40 mg/day such as about 5, 10, 20 or 30 mg/day.

Additionally provided herein are uses of various 5-HT2A serotonin receptor inverse agonists or antagonists to treat a subject by reducing brain levels of Aβ peptides, reducing the rate of accumulation of aggregated Aβ peptides in the brain, and reducing the rate of accumulation of amyloid plaques.

Also provided are uses of 5-HT2A serotonin receptor inverse agonists or antagonists to treat a subject by reducing the rate of accumulation of aggregated beta amyloid in the brain, and reducing the rate of accumulation of amyloid plaques.

In some embodiments the 5-HT2A serotonin receptor inverse agonists may be used in combination with antidepressant medications that elevate synaptic levels of serotonin to treat a subject by reducing brain levels of Aβ peptides, and reducing the rate of accumulation of amyloid plaques.

In some embodiments the 5-HT2A serotonin receptor inverse agonists may be used in combination with 5-HT4 serotonin receptor agonists to treat a subject by reducing brain levels of Aβ peptides, and reducing the rate of accumulation of amyloid plaques.

In some embodiments the 5-HT2A serotonin receptor inverse agonists may be used in combination with antipsychotic medications to treat a subject by reducing brain levels of Aβ peptides, and reducing the rate of accumulation of amyloid plaques.

In some embodiments the 5-HT2A serotonin receptor inverse agonists may be used in combination with beta-secretase 1 (BACE1) inhibitors (or in a composition comprising the 5-HT2A serotonin receptor inverse agonists and a BACE1 inhibitor) to treat a subject by reducing the rate of accumulation of amyloid plaques, or reduce the concentration of Aβ peptides. Examples of BACE1 inhibitors that may be used in combination with the 5-HT2A serotonin receptor inverse agonist, such as pimavanserin, include, but are not limited to AZD3293 (for example at an oral dose of about 20 and about 50 mg, once daily), verubecestat (MK-8931, for example at an oral dose of about 12 mg and about 40 mg, once daily), AMG 520, LY2886721 (for example at a dose of about 15 mg, about 35 mg or about 70 mg), CTS-21166 (for example at a dose of about 7.5 to about 225 mg), and E2609 (for example at an oral dose of about 50 mg, once daily).

In some embodiments the 5-HT2A serotonin receptor inverse agonists, such as pimavanserin, may be used in combination with gamma-secretase inhibitors (or in a composition comprising the 5-HT2A serotonin receptor inverse agonists and a gamma-secretase inhibitor) to treat a subject by reducing the rate of accumulation of amyloid plaques, or reduce the concentration of Aβ peptides.

In some embodiments the HT2A serotonin receptor inverse agonists, such as pimavanserin, may be used in combination with M1 muscarinic acetylcholine receptor agonists (or in a composition comprising the 5-HT2A serotonin receptor inverse agonists and a M1 muscarinic acetylcholine receptor agonist) to treat a subject by reducing the rate of accumulation of amyloid plaques, or reduce the concentration of Aβ peptides. A suitable example of M1 muscarinic acetylcholine receptor agonists is xanomeline (for example at doses of about 75 mg, 150 mg, or 225 mg per day).

In some embodiments the 5-HT2A serotonin receptor inverse agonists, such as pimavanserin, may be used in combination with anti-amyloid beta monoclonal antibodies (or in a composition comprising the 5-HT2A serotonin receptor inverse agonists and anti-amyloid beta monoclonal antibodies) to treat a subject by reducing the rate of accumulation of amyloid plaques, or reduce the concentration of Aβ peptides. Examples of anti-amyloid beta monoclonal antibodies that may be used in combination with the 5-HT2A serotonin receptor inverse agonist include, but are not limited to Aducanumab (BIIB037; for example at a dose of about 1, about 3, about 6 or about 10 mg/kg, intraveneously monthly), Gantenerumab (for example at a dose of about 75 mg, about 105 mg, or about 225 mg, sub-cutaneously every 4 weeks), Crenezumab (for example at a dose of about 15 mg/kg, about 30 mg/kg, or about 60 mg/kg intraveneously once per month),, bapineuzumab (for example at a dose of about 2 mg, about 7 mg, or about 20 mg, sub-cutaneously once per month), solanezumab (LY2062430; for example at a dose of about 400 mg every 4 weeks), and BAN2401 (for example at a dose of about 0.3, about 1, about 2.5, about 3, about 5 or about 10 mg/kg intraveneous every 4 weeks).

In some embodiments the 5-HT2A inverse agonist is a selective 5-HT2A inverse agonist, such as pimavanserin.

In some embodiments of the methods for reducing concentration of an Aβ peptide in a subject provided herein, the methods comprise administering a selective 5-HT2A serotonin receptor inverse agonist or antagonist to the subject, whereby the subject's Aβ peptide concentration is reduced as compared to subject's Aβ peptide concentration prior to the selective 5-HT2A serotonin receptor inverse agonist or antagonist administration. The subject's Aβ peptide concentration can, for example, be that in the subject's blood, plasma, CSF or interstitial fluid. In some embodiments, the Aβ peptide concentration is a concentration in the subject's plasma.

In some embodiments the reduced rate of accumulation may lead to a later onset or slower progression and/or a prevention of a neurodegenerative disease or disorder selected from the group consisting of Parkinson's disease, Alzheimer's disease (AD), Down's syndrome, Huntington's disease, fronto-temporal lobar degeneration associated with protein TDP-43 (FTLD-TDP, Dementia with Lewy bodies (DLB), vascular dementia, Amyotrophic lateral sclerosis (ALS), , Parkinson's disease with MCI, Parkinson's disease with dementia, central amyloid angiopathy and other neurodegenerative related dementias due to changes in the brain caused by [accumulation of amyloid plaques] ageing, disease or trauma; or spinal cord injury. Particular examples are Parkinson's disease, Alzheimer's disease, central amyloid angiopathy, Lewy body disease (LBD) and Down's syndrome.

In certain embodiments of the methods provided herein, a subject administered with a selective 5-HT2A serotonin receptor inverse agonist or antagonist is a subject in need of reducing the rate of accumulation of amyloid plaque. For instance, the subject in need can be a subject with Down's syndrome, a subject with dementia such as senile dementia, a subject with AD (Alzheimer's disease), a subject with MCI (mild cognitive impairment), or an elderly subject, or a subject at risk of developing such a disease.

In yet other embodiments, provided herein are methods for inhibiting aggregation of Aβ peptides in a subject comprising administering a selective 5-HT2A serotonin receptor inverse agonist or antagonist to the subject.

In yet other embodiments, provided herein are methods for inhibiting aggregation of Aβ peptides in a subject comprising administering a selective 5-HT2A serotonin receptor inverse agonist or antagonist, such as those described herein, to the subject.

Some embodiments relate to a compound, a composition or a method for reducing the rate of accumulation of amyloid plaques comprising administering an effective amount of a 5-HT2A serotonin receptor inverse agonist or antagonist, or a pharmaceutically acceptable salt thereof to a subject. The accumulation is generally considered to take place in the brain of the subject, e.g. the in the central nervous system. The subject is generally considered a subject in need of reducing the rate of accumulation of amyloid plaques, reducing the concentration of Aβ peptides (Aβ), or controlling the production and/or elimination of Aβ peptides (Aβ). In some aspects the subject is showing signs of accumulation of amyloid plaques, or increased concentration or production of Aβ peptides (Aβ) prior to being administered the 5-HT2A serotonin receptor inverse agonist or antagonist. The subject may for example be identified by using based on one or more of the following: amyloid plaque imaging using Positron Imaging Tomography (PET); genetic testing for a mutation in the amyloid precursor protein (APP) gene; genetic testing for a gene involved in processing amyloid precursor protein (APP); genetic testing an apolipoprotein E (APOE) ε4 carrier; genetic testing for a strong family history of Alzheimer's disease; genetic testing for trisomic for the amyloid precursor protein (APP) gene; changes in amyloid biomarkers, such as Aβ(1-40), Aβ(1-42), and Aβ(1-x); changes in tau biomarkers, such as total tau (t-tau) and phosphor-tau (p-tau); changes from baseline in total brain, cortical, hippocampal or ventricular volume; and/or the subject has mild cognitive impairment. Alternatively the subject is identified by one or more of the following: detecting amyloid plaque in the subject using Positron Imaging Tomography (PET); determining by genetic testing that the subject has a mutation in the amyloid precursor protein (APP) gene; detecting a mutation on chromosome 21, mutations on chromosome 14 or mutations on chromosome 1 involved in processing amyloid precursor protein (APP); determining by genetic testing that the subject is an apolipoprotein E (APOE) ε4 carrier; determining by genetic testing that the subject has a strong family history of Alzheimer's disease; determining by genetic testing that the subject is trisomic for the amyloid precursor protein (APP) gene; detecting changes in amyloid biomarkers in the subject; detecting changes in tau biomarkers in the subject; detecting changes from baseline in total brain, cortical, hippocampal or ventricular volume in the subject's brain; and/or determining the subject has mild cognitive impairment. The rate of accumulation of the amyloid plaques may be reduced by reducing the production of Aβ, and/or by increasing the elimination of Aβ in the brain, for example the central nervous system, particular examples are reducing the production and or increasing the elimination of Aβ40 and Aβ42.

Some embodiments relate to a composition for reducing the rate of accumulation of amyloid plaques comprising administering an effective amount of a 5-HT2A serotonin receptor inverse agonist or antagonist, or a pharmaceutically acceptable salt thereof and an effective amount of an agent selected from the group containing an antidepressant, an anti-amyloid beta monoclonal antibody, an M1 muscarinic acetylcholine receptor agonist, and a beta-secretase 1 (BACE1) inhibitor to a subject. Examples of antidepressants are selective serotonin reuptake inhibitor (SSRI) or serotonin-norepinephrine reuptake inhibitor (SNRI).

Disclosed herein are data on the effects of selective 5-HT2A inverse agonists on ISF Aβ levels in a mouse model of AD, the APP/PS1 transgenic mouse. These mice develop amyloid plaques in a progressive, age-dependant manner, starting at approximately 4.5 months of age, which are fully formed by 8 -9 months of age, and which display concomitant impairments in cognitive function (Trinchese et al., 2004). The results herein demonstrate that M100,907 (also called volinanserin) robustly lowered levels of Aβ in a dose dependent manner in both young (3 month old, pre-plaque) and old (12 month old, fully formed amyloid plaques) APP/PS1 mice (Example 1). In addition, similar Aβ lowering effects of M100,907 were observed in wild-type mice. Pimavanserin also robustly lowered ISF Aβ in APP/PS1 mice in a dose dependent manner (Example 2), and could do this whether administered in single bolus doses, or as a continuous infusion. Significantly, the Aβ-lowering effects of both M100,907 and pimavanserin were abolished in mice in which the 5-HT2A receptor gene had been deleted (henceforth 5-HT2A KO or 5-HT2A -/- mice, Example 3). These results confirm that the Aβ-lowering effects of M100,907 and pimavanserin depend on their interaction with the 5-HT2A receptor. These results also strongly suggest most, if not all other selective 5-HT2A receptor inverse agonists, including the ones listed herein, should also lower Aβ.

Disclosed here are data on the effects of selective 5-HT2A inverse agonists on ISF Aβ levels in a mouse model of AD, the APP/PS1 transgenic mouse. These mice develop amyloid plaques in a progressive, age-dependant manner, starting at approximately 4.5 months of age, which are fully formed by 8 -9 months of age, and which display concomitant impairments in cognitive function (Trinchese et al., 2004). The results herein demonstrate that M100,907 (also called volinanserin) robustly lowered levels of Aβ in a dose dependent manner in both young (3 month old, pre-plaque) and old (12 month old, fully formed amyloid plaques) APP/PS1 mice (Example 1). In addition, similar Aβ lowering effects of M-100,907 were observed in wild-type mice. Pimavanserin also robustly lowered ISF Aβ in APP/PS1 mice in a dose dependent manner (Example 2), and could do this whether administered in single bolus doses, or as a continuous infusion. Significantly, the Aβ-lowering effects of both M100,907 and pimavanserin were abolished in mice in which the 5-HT2A receptor gene had been deleted (henceforth 5-HT2A KO or 5-HT2A -/- mice, Example 3). These results confirm that the Aβ-lowering effects of M100,907 and pimavanserin depend on their interaction with the 5-HT2A receptor. These results also strongly suggest most, if not all other selective 5-HT2A receptor inverse agonists, including the ones listed herein, should also lower Aβ.

Ultimately, the problem with excess Aβ peptides, for example Aβ40 and Aβ42, is that they can aggregate to form fibrils, which in turn adhere together to form mats, which clump together to finally form plaques called amyloid plaques that accumulate in the brain, damage neurons and contribute to diseases like Alzheimer's disease, Down's syndrome, LBD or central amyloid angiopathy. Thus the ultimate goal of any anti-amyloid treatment is to prevent or reduce the formation of amyloid plaques. To address this, we conducted two experiments. In the first experiment, we administered pimavanserin to 4.5 month old APP/PS1 mice, an age before these mice have developed amyloid plaques, until these mice were 8.5 months old, an age where they have developed significant amyloid plaques. In the second experiment, we administered pimavanserin to 6 month old APP/PS1 mice, an age when these mice have started to develop amyloid plaques, until these mice were 10 months old, an age where they have developed even larger amyloid plaques. The results of the first experiment are shown in Examples 4 and 5, and the results of the second experiment are shown in Examples 6 and 7.

Examples 4 and 6 show the effect of pimavanserin treatment on the accumulation of Aβ40 and Aβ42 peptides in guanidine extracts from hippocampus measured using ELISA. The hippocampus is one of the brain structures critically involved in memory that is impaired by accumulation of amyloid plaque. The guanidine fraction represents the insoluble, aggregated amyloid that comprises amyloid plaques. There was a highly significant reduction in levels of the guanidine fraction of Aβ40 and Aβ42 in the pimavanserin treated mice compared to the vehicle treated mice.

Strikingly, there was not a significant difference in the levels of Aβ40 and Aβ42 between the 'Start' groups (age matched mice sacrificed at the beginning of each experiment) and the pimavanserin treated groups at the end of the experiment suggesting that not only did pimavanserin lower plaque load, but it actually prevented an increase in plaque altogether (Examples 4 and 6). It was also significant that pimavanserin worked well either when administered to mice starting at age 4.5 months, before they start developing plaques, or when administered to mice starting at age 6 months, after they have started developing plaques. Thus pimavanserin may be administered to subjects either prophylactically, or at any point after they start to develop amyloid plaques to halt or slow down further growth of the amyloid plaques. Remarkably, there was a reduction in Aβ40 in male mice in the pimavanserin treated group (age 10 months) compared to the start group (age 6 months) showing pimavanserin may reverse plaque accumulation in some cases (Example 6B).

Examples 5 and 7 show histological staining of hippocampus for amyloid plaque. Consistent with the data presented above, there were highly significant decreases of amyloid plaque in the pimavanserin treated mice compared to the vehicle treated mice. Together, these data demonstrate that selective 5-HT2A receptor inverse agonists, especially pimavanserin, are effective agents for reducing the accumulation of aggregated Aβ40, Aβ42, and amyloid plaque. Significantly, pimavanserin worked well either when administered to mice starting at age 4.5 months, before they start developing plaques, or when administered to mice starting at age 6 months, after they have started developing plaques. Accordingly, pimavanserin may slow down or even prevent human diseases connected to accumulation of amyloid plaque, and may be administered to subjects either prophylactically, or at any point after they start to develop amyloid plaques to halt or slow down further growth of the amyloid plaques.

The CSF contains Aβ and is an accessible compartment which provides a convenient means to assess the effects of drugs on Aβ levels in humans. As shown in Example 8, administration of pimavanserin to 6 month old APP/PS1 mice for 4 months significantly lowered Aβ. Similar results were observed when pimavanserin was administered to 4.5 old APP/PS1 mice for 4 months (not shown). Therefore one could administer pimavanserin to a person in need of a drug to lower Aβ levels and/or reduce the rate of amyloid plaque accumulation, and measure Aβ levels in that person's CSF.

Consequently pimavanserin in some embodiments is administered to a subject, such as a patient at risk of developing any of the above mentioned diseases. In an embodiment pimavanserin is administered to a subject to reduce concentration of Aβ peptides (Aβ), e.g. Aβ40 and/or Aβ42; and/or to reduce the rate of accumulation of aggregated Aβ peptides or amyloid plaque in order to prophylactically prevent or slow down the emergence or progression of one or more diseases connected to accumulation of amyloid plaque e.g. Alzheimer's disease and Down's syndrome.

In an embodiment pimavanserin is administered to a subject prior to the development of substantial symptoms of a disease connected to the accumulation of amyloid plaques, such as Alzheimer's disease and Down's syndrome, wherein the rate of accumulation of amyloid plaques is reduced.

In an embodiment pimavanserin is administered to a subject such as a subject in need, e.g. by showing signs of accumulation of amyloid plaques. Such subjects may be identified using Positron Imaging Tomography (PET), a nuclear medicine neuroimaging tool that can be used to make a picture of amyloid plaque accumulation within a person's brain.

A subject, such as a human person, may be forming amyloid plaque in the brain and therefore at risk of developing a neurodegenerative disease, such as Alzheimer's disease (AD), Parkinson's disease (PD), Down's syndrome or Lewy body disease (LBD).

Furthermore subjects may have mild cognitive impairment, which may indicate onset of accumulation of amyloid plaque, and such persons may benefit from receiving treatments that reduce or prevent the accumulation of amyloid plaque before they are diagnosed with AD. In addition, persons carrying certain mutations, such as mutations in the APP gene, or in genes involved in processing APP such as presenilin 1, or such as APOE ε4 carriers, or persons with a strong family history of AD, or persons who are trisomic for the APP gene may at some point benefit from receiving treatments that reduce or prevent the accumulation of amyloid plaque before they are diagnosed with AD.

Other subjects potentially in need of treatments that reduce or prevent the accumulation of amyloid plaque may be identified by changes in cerebrospinal fluid (CSF) biomarkers t-tau and p-tau or CSF amyloid biomarkers Aβ(1-40), Aβ(1-42), and Aβ(1-x) changes from baseline in total hippocampal volume using volumetric magnetic resonance imaging (vMRI).

Still other subjects that may be particularly in need of treatments that reduce or prevent the rate of accumulation of amyloid plaque may include persons who have any combination of signs of accumulation of amyloid plaque and/or certain genetic mutations and/or certain changes in biomarkers including and/or mild cognitive impairment, but not limited to those described above.

In an embodiment pimavanserin is administered to a subject characterized by said subject showing signs of dementia, e.g. Alzheimer's disease, and wherein the administration reduces the rate of accumulation of amyloid plaques.

In an embodiment pimavanserin is administered to a subject who is identified as having started to develop amyloid plaques by PET scanning, and wherein the administration reduces the rate of accumulation of amyloid plaques.

In an embodiment pimavanserin is administered to a subject who is identified as having mutations on chromosome 21 that cause the formation of abnormal amyloid precursor protein (APP), or in genes involved in processing APP such as mutations on chromosome 14 that cause abnormal presenilin 1 to be made or mutations on chromosome 1 that lead to abnormal presenilin 2, and wherein the administration reduces the rate of accumulation of amyloid plaques.

In an embodiment pimavanserin is administered to a subject who is identified as having one or more alleles of the APOE ε4 gene, and wherein the administration reduces the rate of accumulation of amyloid plaques.

In an embodiment pimavanserin is administered to a subject who is identified as trisomic for the APP gene, and wherein the administration reduces the rate of accumulation of amyloid plaques.

In an embodiment pimavanserin is administered to a subject who is identified as having mild cognitive impairment, and wherein the administration reduces the rate of accumulation of amyloid plaques.

In an embodiment pimavanserin is administered to a subject having a risk of accumulating amyloid plaque. Non-limiting examples include subjects being imaged showing signs of accumulation of amyloid plaque, genetically dispositioned subjects or any one shown to have a risk of accumulating amyloid plaque.

### EXAMPLES

### Example 1. MDL 100,907 (Volinanserin) a selective 5-HT2A antagonist normalizes brain ISF Aβ levels in living wild-type and amyloid precursor protein (APP)/ presenilin1 (PS1) transgenic mice

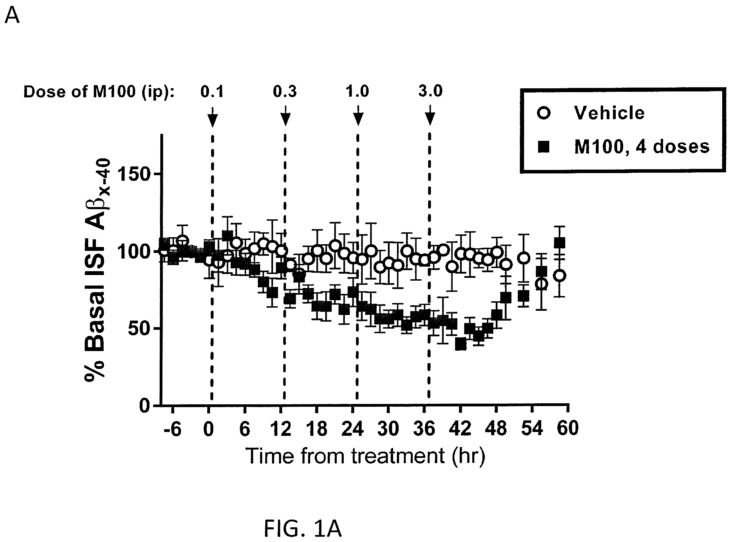

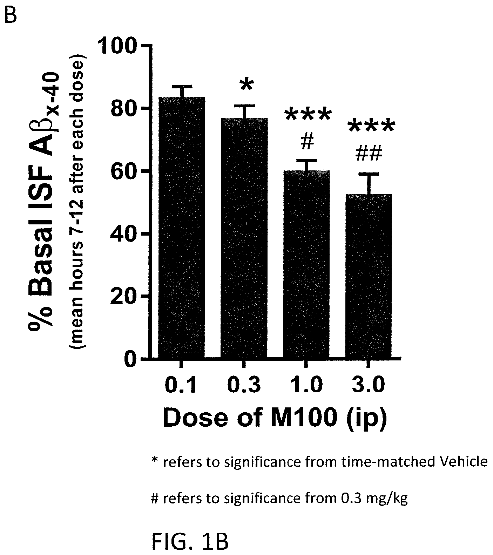

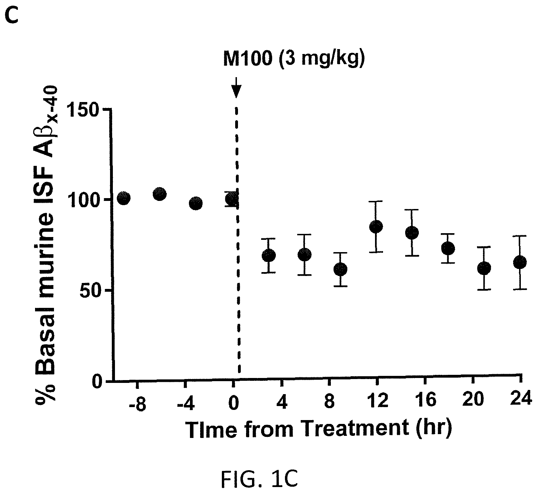

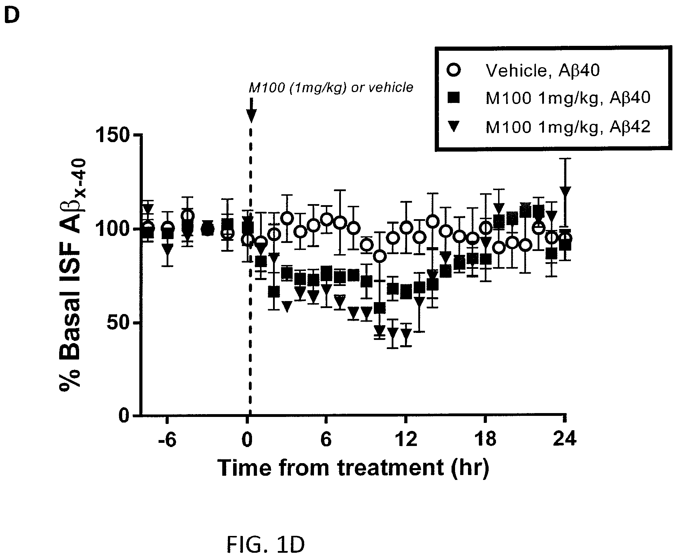

Wild-type or APP/PS1+/- transgenic mice at the indicated ages (equal male and female) were implanted with unilateral hippocampal microdialysis probes at stereotaxic coordinates (bregma - 3.1mm, 2.5m lateral to midline, and 1.2mm below dura at a 12º angle) as described (Cirrito et al. 2003; 2011). These probes contain a 38kDa MWCO semi-permeable membrane and sample Aβ specifically from within the brain ISF. ISF Aβ was measured every 60-90 minutes for up to 72 hours after probe was inserted into the brain. Throughout each study mice was housed in RaTurn caging systems to provide freedom of movement and ad lib food and water while attached the microdialysis equipment. After probe insertion, animals were allowed to recover for 6 hours in order for the brain to recover and the blood-brain barrier to reform. Microdialysis probe insertion was similar to the procedure described by Cirrito et al. 2003 and 2011. From hours 6-60 after microdialysis probe insertion, ISF Aβ levels were relatively constant which provides the ideal experimental window. After recovery, each mouse had baseline ISF Aβ levels sampled every hour over a 6 hour period followed by administration of either vehicle (saline, pH 6.0 or MDL 100,907. Following drug administration, ISF Aβ was measured every 60-90 minutes for an additional 48 hours. At the end of each experiment ISF Aβ40 was immediately measured by sandwich ELISA. Animals were sacrificed, brains removed, then processed for histological verification of probe placement. Figure 1 discloses the results of Example 1 (MDL 100,907 (M100), a selective 5-HT2A Serotonin Receptor Inverse Agonist on ISF levels of Aβ40 and Aβ42 in wild-type, and in young and aged APP/PS1^{+/-} Transgenic Mice). A) Young (2 to 3 month) APP/PS1 mice. B) dose-response in young (2 to 3 month) APP/PS1 mice. "*" significantly different from time-matched vehicle control."#" significantly different from 0.3 mg/kg. C) Wild-type C57BI6 mice. D) Aged (9 to 12 month) APP/PS1^{+/-} transgenic mice.

### Example 2. Pimavanserin, a selective 5-HT2A antagonist normalizes brain ISF Aβ levels in living APP/PS1+/- transgenic mice

APP/PS1+/- transgenic mice at the indicated ages (equal male and female) were implanted with unilateral hippocampal microdialysis probes at stereotaxic coordinates (bregma -3.1mm, 2.5m lateral to midline, and 1.2mm below dura at a 12º angle) as described (Cirrito et al. 2003 and 2011) and ISF Aβ levels measured as described in Example 1. Vehicle was phosphate buffered saline (PBS), pH 7.4. Figure 2 discloses the results of Example 2 (pimavanserin, a selective 5-HT2A Serotonin Receptor Inverse Agonist on ISF levels of Aβ40 and Aβ42 in wild-type, and in young and aged APP/PS1^{+/-} Transgenic Mice). A) Dose-response. B) Dose-response means. C) Dose-response by gender. D) Osmotic pump administration. E) Comparison of mean responses. "*" significantly different between groups indicated by brackets.

### Example 3. Pimavanserin and MDL 100,907, selective 5-HT2A antagonists, normalize brain ISF Aβ levels in living wild-type mice, but not in 5-HT2A -/- mice.

Male and female wild-type or 5-HT2A -/- transgenic mice (mice with the 5-HT2A receptor gene deleted), 2.5 to 3 months of age were implanted with unilateral hippocampal microdialysis probes as described in Example 1. After recovery, each mouse had baseline ISF Aβ levels sampled every 3 hours over a 10 hour period followed by administration of either vehicle (saline, pH 6.0) or drug. Following drug administration, ISF Aβ was measured every 3 hours for an additional 24 hours. A) Pimavanserin, 1 mg/kg. B) MDL 100,907, 3 mg/kg.

### Example 4. Chronic administration of pimavanserin to 4.5 month old APP/PS1+/- transgenic mice for 4 months reduces levels of Aβ40 and Aβ42 in hippocampal extracts.

Male and Female APP/PS1+/- transgenic mice, age 4.5 months, were implanted subcutaneously through an incision on the back with Alzet osmotic minipumps (Model #2006) containing either vehicle (water) or drug solution (25 mg/ml pimavanserin in water). Incisions were closed by suture or staple to insure the pumps remained in place. Every 5 weeks pumps were removed and new full pumps implanted in the same manner at a different location on the back to provide continuous drug delivery over the 4 month period. In parallel, age and gender matched cohorts of APP/PS1+/- transgenic mice were sacrificed so that amyloid plaque and Aβ levels at the start of treatment could be compared to mice at the end of treatment. The model #2006 pump delivers 0.15 µl/hr, thus for a 30 g mouse, this rate delivers 3 mg/kg/day of pimavanserin for up to 6 weeks. Every 5 weeks, pumps were replaced with new full pumps to provide uninterrupted delivery of pimavanserin over a 4 month period. At the end of the 4 month treatment period, mice were sacrificed, brains removed, and divided into hemispheres. One hemisphere per mouse was processed for guanidine extraction and biochemical analysis by ELISA of Aβ40 and Aβ42 levels in hippocampus as described in Cirrito et al., 2011, with the 5 Molar Guanidine extracts representing the insoluble fractions of Aβ40 and Aβ42. The insoluble fraction is believed to represent the harmful amyloid plaque deposits that are characteristic of Alzheimer's disease. The other hemisphere was used to perform histological staining and analysis of amyloid plaque growth. Start indicates the age and gender matched cohorts sacrificed at the start of the study. Guan indicates 5 Molar Gaunidine. A) 5M Gaunidine fraction of Aβ40 and Aβ42, all genders. B) 5M Guanidine fraction, male and female. "*" Pim group significantly different from vehicle control. "#" Pim group significantly different from Start group. "ns" Pim group not significantly different from Start group.

### Example 5. Chronic administration of pimavanserin to 4.5 month old APP/PS1+/- transgenic mice for 4 months reduces amyloid plaque load.

Male and Female APP/PS1+/- transgenic mice, age 4.5 months, were implanted with Alzet osmotic minipumps and treated as described in Example 4. At the end of the 4 month treatment period, mice were sacrificed, brains removed, and divided into hemispheres. One hemisphere was used to perform histological staining and analysis of amyloid plaque load. A) Plaque load, all genders. B) Plaque load, male and female. "*" Pim group significantly different from vehicle control.

### Example 6. Chronic administration of pimavanserin to 6 month old APP/PS1+/- transgenic mice for 4 months reduces levels of Aβ40 and Aβ42 in hippocampal extracts.

Male and Female APP/PS1+/- transgenic mice, age 6 months, were implanted with Alzet osmotic minipumps and treated as described in Example 4. At the end of the 4 month treatment period, mice were sacrificed, brains removed, and divided into hemispheres. In parallel, age and gender matched cohorts of APP/PS1+/- transgenic mice were sacrificed so that amyloid plaque and Aβ levels at the start of treatment could be compared to mice at the end of treatment. Start indicates the age and gender matched cohorts sacrificed at the start of the study. Guan indicates 5 Molar Gaunidine. A) 5M Gaunidine fraction of Aβ40 and Aβ42, all genders. B) F) 5M Guanidine fraction of Aβ40 and Aβ42, male and female. "*" Pim group significantly different from vehicle control. "ns" Pim group not significantly different from Start group.Example 7. Chronic administration of pimavanserin to 6 month old APP/PS1+/- transgenic mice for 4 months reduces amyloid plaque growth.

Male and Female APP/PS1+/- transgenic mice, age 6 months, were implanted with Alzet osmotic minipumps and treated as described in Example 4. At the end of the 4 month treatment period, mice were sacrificed, brains removed, and divided into hemispheres. One hemisphere was used to perform histological staining and analysis of amyloid plaque load. A) Plaque load, all genders. B) Plaque load, male and female. "*" Pim group significantly different from vehicle control.

### Example 8. Chronic administration of pimavanserin to 6 month old APP/PS1+/- transgenic mice for 4 months reduces levels of Aβ40 and Aβ42 in CSF.

Male and Female APP/PS1+/- transgenic mice, age 6 months, were implanted with Alzet osmotic minipumps and treated as described in Example 4. At the end of the 4 month treatment period, mice were sacrificed, CSF removed using the method of DeMattos et al., 2002 (see page 230, Materials and Methods), and Aβ40 and Aβ42 levels analyzed by ELISA. A) Aβ40 and Aβ42 in CSF, all genders. B) Aβ40 and Aβ42 in CSF, male and female. "*" Pim group significantly different from vehicle control.

Accordingly the disclosed examples support a 5-HT2A serotonin receptor inverse agonist or antagonist, such as pimavanserin and volinanserin can reduce the rate of accumulation of amyloid plaques in a subject, as well as reducing the concentration of Aβ peptides (Aβ) in a subject.

### REFERENCES

Burstein ES, Piu F, Ma JN, Weissman JT, Currier EA, Nash NR, Weiner DM, Spalding TA, Schiffer HH, Del Tredici AL, Brann MR. (2006) Integrative functional assays, chemical genomics and high throughput screening: harnessing signal transduction pathways to a common HTS readout. Curr Pharm Des. 12:1717-29.
Caccamo A, Oddo S, Billings LM, Green KN, Martinez-Coria H, Fisher A, LaFerla FM. (2006) M1 receptors play a central role in modulating AD-like pathology in transgenic mice. Neuron 49:671e682.
Cirrito JR, May PC, O'Dell MA, Taylor JW, Parsadanian M, Cramer JW, Audia JE, Nissen JS, Bales KR, Paul SM, DeMattos RB, Holtzman DM. (2003) In vivo assessment of brain interstitial fluid with microdialysis reveals plaque-associated changes in amyloid-beta metabolism and half-life. J Neurosci. Oct 1;23(26):8844-53.
Cirrito JR, Disabato BM, Restivo JL, Verges DK, Goebel WD, Sathyan A, Hayreh D, D'Angelo G, Benzinger T, Yoon H, Kim J, Morris JC, Mintun MA, ShelineYI. (2011) Serotonin signaling is associated with lower amyloid-β levels and plaques in transgenic mice and humans. Proc Natl Acad SciUSA 108:14968-73.
Cochet M, Donneger R, Cassier E, Gaven F, Lichtenthaler SF, Marin P, Bockaert J, Dumuis A, Claeysen S. (2013) 5-HT4 receptors constitutively promote the non-amyloidogenic pathway of APP cleavage and interact with ADAM10.ACS ChemNeurosci. Jan 16;4(1):130-40.
Davis AA, Fritz JJ, Wess J, Lah JJ, Levey Al (2010) Deletion of M1 muscarinic acetylcholine receptors increases amyloid pathology in vitro and in vivo. J Neurosci 30: 4190e4196
DeMattos RB, Bales KR, Parsadanian M, O'Dell MA, Foss EM, Paul SM, Holtzman DM. Plaque-associated disruption of CSF and plasma amyloid-beta (Abeta) equilibrium in a mouse model of Alzheimer's disease. J Neurochem. 2002 Apr;81(2):229-36.
Eckols K, Bymaster FP, Mitch CH, Shannon HE, Ward JS, DeLapp NW. (1995) The muscarinic M1 agonist xanomeline increases soluble amyloid precursor protein release from Chinese hamster ovary-ml cells. Life Sci 57: 1183e1190.
Hacksell U, Burstein ES, McFarland K, Mills RG, Williams H. (2014) On the discovery and development of pimavanserin: a novel drug candidate for Parkinson's psychosis. Neurochem Res. Oct;39(10):2008-2017.
Kamenetz F, Tomita T, Hsieh H, Seabrook G, Borchelt D, Iwatsubo T, Sisodia S, Malinow R. APP processing and synaptic function. Neuron. 2003 Mar 27;37(6):925-37.
Khanahmadi M, Farhud DD, Malmir M. (2015) Genetic of Alzheimer's Disease: A Narrative Review Article. Iran J Public Health. Jul;44(7):892-901.
Lomakin A, Teplow DB, Kirschner DA, Benedek GB (1997) Kinetic theory of fibrillogenesis of amyloid beta-protein. Proc Natl Acad Sci USA 94:7942e7947.
Morris JC, Price AL (2001) Pathologic correlates of nondemented aging, mild cognitive impairment, and early-stage Alzheimer's disease. J MolNeurosci 17:101e118
Nitsch RM, Slack BE, Wurtman RJ, Growdon JH (1992) Release of Alzheimer amyloid precursor derivatives stimulated by activation of muscarinic acetylcholine receptors. Science 258:304e307.
Nitsch RM, Deng M, Tennis M, Schoenfeld D, Growdon JH (2000) The selective muscarinic M1 agonist AF102B decreases levels of total Abeta in cerebrospinal fluid of patients with Alzheimer's disease. Ann Neurol 48:913e918.
Pearson HA and Peers C. (2006) Physiological roles for amyloid β peptides. J Physiol. 575(Pt 1): 5-10.
Schupf N, Patel B, Pang D, Zigman WB, Silverman W, Mehta PD, Mayeux R. (2007) Elevated plasma beta-amyloid peptide Abeta(42) levels, incident dementia, and mortality in Down syndrome. Arch Neurol. Jul 64(7):1007-1013.
Sheline YI, West T, Yarasheski K, Swarm R, Jasielec MS, Fisher JR, Ficker WD, Yan P, Xiong C, Frederiksen C, Grzelak MV, Chott R, Bateman RJ, Morris JC, Mintun MA, Lee JM, Cirrito JR. (2014) An antidepressant decreases CSF Aβ production in healthy individuals and in transgenic AD mice. Sci Transl Med. May 14;6(236):236re4.
Shen F, Smith JA, Chang R, Bourdet DL, Tsuruda PR, Obedencio GP, Beattie DT. (2011) 5-HT(4) receptor agonist mediated enhancement of cognitive function in vivo and amyloid precursor protein processing in vitro: A pharmacodynamic and pharmacokinetic assessment. Neuropharmacology 61:69e79.
St George-Hyslop PH, Morris JC (2008) Will anti-amyloid therapies work for Alzheimer's disease? Lancet 372:180e182.
Tesseur I, Pimenova AA, Lo AC, Ciesielska M, Lichtenthaler SF, De Maeyer JH, Schuurkes JA, D'Hooge R, De Strooper B. (2013) Chronic 5-HT4 receptor activation decreases Aβ production and deposition in hAPP/PS1 mice.Neurobiol Aging. Jul 34(7):1779-89.
Trinchese F, Liu S, Battaglia F, Walter S, Mathews PM, Arancio O. (2004) Progressive age-related development of Alzheimer-like pathology in APP/PS1 mice. Ann Neurol. Jun 55(6):801-814.

## Claims

1. Selective 5-HT2A serotonin receptor inverse agonist or antagonist, or a pharmaceutically acceptable salt thereof, for use in a method of delaying the onset and/or preventing Alzheimer's disease (AD) by reducing the concentration of Aβ peptides (Aβ) in a subject, wherein the concentration is in the brain of the subject; said method comprising administering an effective amount of a selective 5-HT2A serotonin receptor inverse agonist or antagonist or a pharmaceutically acceptable salt thereof to the subject in need thereof.

2. The selective 5-HT2A serotonin receptor inverse agonist or antagonist, or a pharmaceutically acceptable salt thereof for use according to claim 1, wherein, prior to being administered to the subject, said subject is identified by one or more of the following:
amyloid plaque imaging using Positron Imaging Tomography (PET);
genetic testing for a mutation in the amyloid precursor protein (APP) gene;
genetic testing for a gene involved in processing amyloid precursor protein (APP);
genetic testing an apolipoprotein E (APOE) ε4 carrier;
genetic testing for trisomy for the amyloid precursor protein (APP) gene;
changes in amyloid biomarkers;
changes in tau biomarkers;
reduction from baseline in total whole brain, cortical or hippocampal volume;
increase in brain ventricular volume; and/or
the subject has mild cognitive impairment.

3. The selective 5-HT2A serotonin receptor inverse agonist or antagonist, or a pharmaceutically acceptable salt thereof for use according to claim 1, wherein the subject has increased risk of developing Alzheimer's disease, wherein the increased risk of developing Alzheimer's disease or dementia is associated with diabetes, high blood pressure, obesity, smoking, depression, cognitive inactivity, low education, low physical inactivity, excessive alcohol intake, or subjects who experience severe or repeated head injuries.

4. The selective 5-HT2A serotonin receptor inverse agonist or antagonist, or a pharmaceutically acceptable salt thereof for use according to any one of claims 1-3, the method further comprising identifying a subject to be administered the selective 5-HT2A inverse agonist or antagonist, wherein the subject is identified by one or more of the following:
detecting amyloid plaque imaging in the subject using Positron Imaging Tomography (PET);
determining by genetic testing that the subject has a mutation in the amyloid precursor protein (APP) gene;
detecting a mutation on chromosome 21, mutations on chromosome 14 or mutations on chromosome 1 involved in processing amyloid precursor protein (APP);
determining by genetic testing that the subject is an apolipoprotein E (APOE) ε4 carrier;
determining by genetic testing that the subject has a strong family history of Alzheimer's disease;
determining by genetic testing that the subject is trisomic for the amyloid precursor protein (APP) gene;
detecting changes in amyloid biomarkers in the subject;
detecting changes in tau biomarkers in the subject;
detecting reduction from baseline in total whole brain, cortical or hippocampal volume;
detecting increase in brain ventricular volume; and/or
determining that the subject has mild cognitive impairment.

5. The selective 5-HT2A serotonin receptor inverse agonist or antagonist, or a pharmaceutically acceptable salt thereof according to any one of claims 1-4, wherein the selective 5-HT2A serotonin receptor inverse agonist or antagonist is a selective 5-HT2A serotonin receptor inverse agonist, preferably selected from the group consisting of volinanserin (MDL 100,907), eplivanserin (SR-46349B), pruvanserin, pimavanserin (ACP-103, Nuplazid™), glemanserin, nelotanserin (APD125), ITI-007 and Temanogrel, more preferably pimavanserin (ACP-103, Nuplazid™) and Volinanserin (MDL 100,907).

6. The selective 5-HT2A serotonin receptor inverse agonist or antagonist, or a pharmaceutically acceptable salt thereof according to any one of claims 1-5, wherein the selective 5-HT2A serotonin receptor inverse agonist or antagonist is the selective 5-HT2A serotonin receptor inverse agonist pimavanserin (ACP-103, Nuplazid™).

7. A composition comprising a selective 5-HT2A serotonin receptor inverse agonist or antagonist, or a pharmaceutically acceptable salt thereof, and an effective amount of an agent selected from the group consisting of an antidepressant, preferably a selective serotonin reuptake inhibitor (SSRI) or serotonin-norepinephrine reuptake inhibitor (SNRI), an M1 muscarinic acetylcholine receptor agonist, a 5-HT4 serotonin receptor agonist, an anti-amyloid beta monoclonal antibody, and a beta-secretase 1 (BACE1) inhibitor for use in a method according to any one of claims 1 to 6.

8. The selective 5-HT2A serotonin receptor inverse agonist or antagonist, or a pharmaceutically acceptable salt thereof for use according to claim 2 or 4, wherein the tau or amyloid biomarkers are selected from the group consisting of total tau (t-tau) and phosphor-tau (p-tau), Aβ(1-40), Aβ(1-42), and Aβ(1-x) biomarkers.

9. The selective 5-HT2A serotonin receptor inverse agonist or antagonist, or a pharmaceutically acceptable salt thereof for use according to any one of claims 2-8, wherein the changes in amyloid biomarkers and tau biomarkers are in cerebrospinal fluid (CSF) or interstitial fluid (ISF) or plasma.

10. The selective 5-HT2A serotonin receptor inverse agonist or antagonist, or a pharmaceutically acceptable salt thereof for use according to any one of claims 1 to 9, wherein the subject is identified by amyloid plaque imaging using Positron Imaging Tomography (PET) of the brain of the subject; or the subject is identified by mutations on chromosome 21, mutations on chromosome 14 or mutations on chromosome 1.

11. The selective 5-HT2A serotonin receptor inverse agonist or antagonist, or a pharmaceutically acceptable salt thereof for use according to any one of claims 1-10, wherein the Aβ peptides are selected from the group consisting of Aβ38, Aβ40, Aβ42 and Aβ43, preferably from Aβ40 and Aβ42.

## Patentansprüche

1. Selektiver inverser 5-HT2A-Serotoninrezeptor-Agonist oder -Antagonist oder pharmazeutisch annehmbares Salz davon zur Verwendung bei einem Verfahren zum Verzögern des Ausbruchs und/oder zum Verhindern der Alzheimer-Krankheit (AD) durch Verringern der Konzentration von Aß-Peptiden (Aß) bei ein Subjekt, wobei die Konzentration im Gehirn des Subjekts vorliegt; wobei das Verfahren das Verabreichen einer wirksamen Menge eines selektiven inversen 5-HT2A-Serotoninrezeptor-Agonisten oder -Antagonisten oder eines pharmazeutisch verträglichen Salzes davon an das Subjekt, das dies benötigt, umfasst.

2. Selektiver inverser 5-HT2A-Serotoninrezeptor-Agonist oder -Antagonist oder pharmazeutisch annehmbares Salz davon zur Verwendung nach Anspruch 1, wobei das Subjekt vor der Verabreichung an das Subjekt durch eines oder mehrere der folgenden Merkmale identifiziert wird:
Amyloid-Plaque-Bildgebung mittels Positronen-Emissions-Tomographie (PET);
Gentests für eine Mutation im Amyloid-Vorläuferprotein(APP)-Gen;
Gentests für ein Gen, das an der Verarbeitung von Amyloid-Vorläuferprotein (APP) beteiligt ist;
Gentests eines Apolipoprotein-E(APOE)-ε4-Trägers;
Gentests auf Trisomie für das Amyloid-Vorläuferprotein(APP)-Gen;
Veränderungen der Amyloid-Biomarker;
Veränderungen der Tau-Biomarker;
Verringerung des Gesamtvolumens des Gehirns, der Kortikalis oder des Hippocampus gegenüber dem Ausgangswert;
Zunahme des ventrikulären Volumens des Gehirns; und/oder bei dem Subjekt vorliegende leichte kognitive Beeinträchtigung.

3. Selektiver inverser 5-HT2A-Serotoninrezeptor-Agonist oder -Antagonist oder pharmazeutisch annehmbares Salz davon zur Verwendung nach Anspruch 1, wobei das Subjekt ein erhöhtes Risiko für die Entwicklung einer Alzheimer-Krankheit aufweist, wobei das erhöhte Risiko für die Entwicklung einer Alzheimer-Krankheit oder Demenz mit Diabetes, Bluthochdruck, Fettleibigkeit, Rauchen, Depressionen, kognitiver Inaktivität, geringer Bildung, geringer körperlicher Inaktivität, übermäßigem Alkoholkonsum oder bei Personen mit schweren oder wiederholten Kopfverletzungen in Verbindung steht.

4. Selektiver inverser 5-HT2A-Serotoninrezeptor-Agonist oder -Antagonist oder pharmazeutisch annehmbares Salz davon zur Verwendung nach einem der Ansprüche 1-3, wobei das Verfahren ferner das Identifizieren eines Subjekts umfasst, dem der selektive inverse 5-HT2A-Agonist oder -Antagonist verabreicht werden soll, wobei das Subjekt durch eines oder mehrere der folgenden Merkmale identifiziert wird:
Nachweisen von Amyloid-Plaque-Bildgebung mittels Positronen-Emissions-Tomographie (PET);
Bestimmen, durch Gentests, dass bei dem Subjekt eine Mutation im Amyloid-Vorläuferprotein(APP)-Gen vorliegt;
Nachweisen einer Mutation auf Chromosom 21, von Mutationen auf Chromosom 14 oder Mutationen auf Chromosom 1, die an der Verarbeitung von Amyloid-Vorläuferprotein (APP) beteiligt sind;
Bestimmen, durch Gentests, dass das Subjekt ein Apolipoprotein-E(APOE)-ε4-Träger ist;
Bestimmen, durch Gentests, dass bei dem Subjekt Alzheimer-Krankheit in der Familienanamnese deutlich vorliegt;
Bestimmen, durch Gentests, dass das Subjekt für das Amyloid-Vorläuferprotein(APP)-Gen trisomisch ist;
Nachweisen von Veränderungen der Amyloid-Biomarker bei dem Subjekt;
Nachweisen von Veränderungen der Tau-Biomarker bei dem Subjekt;
Nachweisen einer Verringerung des Gesamtvolumens des Gehirns, der Kortikalis oder des Hippocampus gegenüber dem Ausgangswert;
Nachweisen einer Zunahme des ventrikulären Volumens des Gehirns; und/oder
Nachweisen, dass bei dem Subjekt eine leichte kognitive Beeinträchtigung vorliegt.

5. Selektiver inverser 5-HT2A-Serotoninrezeptor-Agonist oder -Antagonist oder pharmazeutisch annehmbares Salz davon nach einem der Ansprüche 1-4, wobei der selektive inverse 5-HT2A-Serotoninrezeptor-Agonist oder -Antagonist ein selektiver inverser 5-HT2A-Serotoninrezeptor-Agonist oder -Antagonist ist, ausgewählt aus der Gruppe bestehend aus Volinanserin (MDL 100, 907), Eplivanserin (SR-46349B), Pruvanserin, Pimavanserin (ACP-103, Nuplazid™), Glemanserin, Nelotanserin (APD125), ITI-007 und Temanogrel, stärker bevorzugt Pimavanserin (ACP-103, Nuplazid™) und Volinanserin (MDL 100, 907).

6. Selektiver inverser 5-HT2A-Serotoninrezeptor-Agonist oder -Antagonist oder pharmazeutisch annehmbares Salz davon nach einem der Ansprüche 1-5, wobei der selektive inverse 5-HT2A-Serotoninrezeptor-Agonist oder -Antagonist der selektive inverse 5-HT2A-Serotoninrezeptor-Agonist oder -Antagonist Pimavanserin (ACP-103, Nuplazid™) ist.

7. Zusammensetzung, umfassend einen selektiven inversen 5-HT2A-Serotoninrezeptor-Agonisten oder -Antagonisten oder ein pharmazeutisch verträgliches Salz davon und eine wirksame Menge eines Mittels, ausgewählt aus der Gruppe bestehend aus einem Antidepressivum, vorzugsweise einem selektiven Serotonin-Wiederaufnahmehemmer (SSRI) oder einem Serotonin-Noradrenalin-Wiederaufnahmehemmer (SNRI), einem M1-Muskarin-Acetylcholinrezeptor-Agonisten, einem 5-HT4-Serotoninrezeptor-Agonisten, einem monoklonalen Anti-Amyloid-Beta-Beta-Antikörper und einem Beta-Sekretase-1(BACE1)-Hemmer zur Verwendung in einem Verfahren nach einem der Ansprüche 1 bis 6.

8. Selektiver inverser 5-HT2A-Serotoninrezeptor-Agonist oder -Antagonist oder pharmazeutisch verträgliches Salz davon zur Verwendung nach Anspruch 2 oder 4, wobei die Tau- oder Amyloid-Biomarker ausgewählt sind aus der Gruppe bestehend aus Gesamt-Tau(t-Tau)- und Phosphor-Tau(p-Tau)-, Aβ(1-40)-, Aβ(1-42)- und Aβ(1-x)-Biomarkern.

9. Selektiver inverser 5-HT2A-Serotoninrezeptor-Agonist oder -Antagonist oder pharmazeutisch annehmbares Salz davon zur Verwendung nach einem der Ansprüche 2-8, wobei die Änderungen der Amyloid-Biomarker und der Tau-Biomarker in Cerebrospinalflüssigkeit (CSF) oder interstitieller Flüssigkeit (ISF) oder Plasma vorliegen.

10. Selektiver inverser 5-HT2A-Serotoninrezeptor-Agonist oder -Antagonist oder pharmazeutisch annehmbares Salz davon zur Verwendung nach einem der Ansprüche 1 bis 9, wobei das Subjekt durch Amyloid-Plaque-Bildgebung unter Verwendung von Positronen-Emissions-Tomographie (PET) des Gehirns des Subjekts identifiziert wird; oder das Subjekt wird durch Mutationen auf Chromosom 21, Mutationen auf Chromosom 14 oder Mutationen auf Chromosom 1 identifiziert.

11. Selektiver inverser 5-HT2A-Serotoninrezeptor-Agonist oder -Antagonist oder pharmazeutisch annehmbares Salz davon zur Verwendung nach einem der Ansprüche 1-10, wobei die Aß-Peptide ausgewählt sind aus der Gruppe bestehend aus Aβ38, Aβ40, Aβ42 und Aβ43, vorzugsweise aus Aβ40 und Aβ42.

## Revendications

1. Agoniste ou antagoniste inverse sélectif des récepteurs de sérotonine 5-HT2A, ou sel pharmaceutiquement acceptable de celui-ci, pour utilisation dans un procédé de retardement de l'apparition et/ou de prévention de la maladie d'Alzheimer (MA) par réduction de la concentration de peptides Aβ (Aβ) chez un sujet, où la concentration est dans le cerveau du sujet ; ledit procédé comprenant l'administration d'une quantité efficace d'un agoniste ou antagoniste inverse sélectif des récepteurs de sérotonine 5-HT2A ou un sel pharmaceutiquement acceptable de celui-ci au sujet en ayant besoin.

2. Agoniste ou antagoniste inverse sélectif des récepteurs de sérotonine 5-HT2A, ou sel pharmaceutiquement acceptable de celui-ci pour utilisation selon la revendication 1, où, avant d'être administré au sujet, ledit sujet est identifié par un ou plusieurs des suivants :
imagerie des plaques amyloïdes chez le sujet au moyen de la tomographie par émission de positons (TEP) ;
des tests génétiques pour une mutation dans le gène de la protéine précurseur d'amyloïde (APP) ;
des tests génétiques pour un gène impliqué dans le traitement de la protéine précurseur d'amyloïde (APP) ;
des tests génétiques pour un support d'apolipoprotéine E (APOE) ε4 ;
des tests génétiques pour la trisomie pour le gène de la protéine précurseur d'amyloïde (APP) ;
des changements de biomarqueurs d'amyloïde ;
des changements de biomarqueurs de tau ;
la réduction par rapport aux valeurs de référence dans le cerveau entier total, du volume cortical ou hippocampique ;
l'augmentation du volume ventriculaire cérébral ; et/ou le sujet présente un trouble cognitif léger.

3. Agoniste ou antagoniste inverse sélectif des récepteurs de sérotonine 5-HT2A, ou sel pharmaceutiquement acceptable de celui-ci pour utilisation selon la revendication 1, où le sujet présente un risque accru de développer la maladie d'Alzheimer, où le risque accru de développer la maladie d'Alzheimer ou une démence est associé au diabète, à l'hypertension artérielle, à l'obésité, au tabagisme, à la dépression, à une inactivité cognitive, à une faible éducation, à une faible activité physique, à l'abus d'alcool, ou des sujets qui présentent des lésions sévères ou répétées de la tête.

4. Agoniste ou antagoniste inverse sélectif des récepteurs de sérotonine 5-HT2A, ou sel pharmaceutiquement acceptable de celui-ci pour utilisation selon l'une quelconque des revendications 1 à 3, le procédé comprenant en outre l'identification d'un sujet auquel doit être administré l'agoniste ou antagoniste inverse sélectif de 5-HT2A, où le sujet est identifié par l'un ou plusieurs des suivants :
détection d'imagerie des plaques amyloïdes chez le sujet au moyen de la tomographie par émission de positons (TEP) ;
la détermination par des tests génétiques du fait que le sujet présente une mutation dans le gène de la protéine précurseur d'amyloïde (APP) ;
la détection d'une mutation sur le chromosome 21, de mutations sur le chromosome 14 ou de mutations sur le chromosome 1 impliquées dans le traitement de la protéine précurseur d'amyloïde (APP) ;
la détermination par des tests génétiques du fait que le sujet est un support d'apolipoprotéine E (APOE) ε4 ;
la détermination par des tests génétiques du fait que le sujet présente des antécédents familiaux significatifs de la maladie d'Alzheimer ;
la détermination par des tests génétiques du fait que le sujet est trisomique pour le gène de la protéine précurseur d'amyloïde (APP) ;
la détection de changements de biomarqueurs d'amyloïde chez le sujet ;
la détection de changements de biomarqueurs de tau dans le sujet ;
la détection d'une réduction par rapport aux valeurs de référence dans le cerveau entier total, du volume cortical ou hippocampique ;
la détection de l'augmentation du volume ventriculaire cérébral ; et/ou
la détermination du fait que le sujet présente un trouble cognitif léger.

5. Agoniste ou antagoniste inverse sélectif des récepteurs de sérotonine 5-HT2A, ou sel pharmaceutiquement acceptable de celui-ci selon l'une quelconque des revendications 1 à 4, où l'agoniste ou antagoniste inverse sélectif des récepteurs de sérotonine 5-HT2A est un agoniste inverse sélectif des récepteurs de sérotonine 5-HT2A, de préférence choisi dans le groupe constitué des volinansérine (MDL 100 907), éplivansérine (SR-46349B), pruvansérine, pimavansérine (ACP-103, Nuplazid™), glémansérine, nélotansérine (APD125), ITI-007 et Temanogrel, plus préférablement la pimavansérine (ACP-103, Nuplazid™) et la volinansérine (MDL 100 907).

6. Agoniste ou antagoniste inverse sélectif des récepteurs de sérotonine 5-HT2A, ou sel pharmaceutiquement acceptable de celui-ci selon l'une quelconque des revendications 1 à 5, où l'agoniste ou antagoniste inverse sélectif des récepteurs de sérotonine 5-HT2A est l'agoniste inverse sélectif des récepteurs de sérotonine 5-HT2A pimavansérine (ACP-103, Nuplazid™).

7. Composition comprenant un agoniste ou antagoniste inverse sélectif des récepteurs de sérotonine 5-HT2A, ou un sel pharmaceutiquement acceptable de celui-ci, et une quantité efficace d'un agent choisi dans le groupe constitué d'un antidépresseur, de préférence un inhibiteur sélectif de la recapture de la sérotonine (ISRS) ou un inhibiteur de la recapture de la sérotonine et de la noradrénaline (IRSN), un agoniste des récepteurs muscariniques d'acétylcholine M1, un agoniste des récepteurs de sérotonine 5-HT4, un anticorps monoclonal anti-bêta-amyloïde et un inhibiteur de bêta-sécrétase 1 (BACE1) pour utilisation dans un procédé selon l'une quelconque des revendications 1 à 6.

8. Agoniste ou antagoniste inverse sélectif des récepteurs de sérotonine 5-HT2A, ou sel pharmaceutiquement acceptable de celui-ci pour utilisation selon la revendication 2 ou 4, où les biomarqueurs de tau ou d'amyloïde sont choisis dans le groupe constitué des biomarqueurs tau total (t-tau) et phospho-tau (p-tau), Aβ(1-40), Aβ(1-42), et Aβ(1-x).

9. Agoniste ou antagoniste inverse sélectif des récepteurs de sérotonine 5-HT2A, ou sel pharmaceutiquement acceptable de celui-ci pour utilisation selon l'une quelconque des revendications 2 à 8, où les changements de biomarqueurs d'amyloïde et de biomarqueurs de tau sont dans le liquide céphalo-rachidien (LCR) ou le liquide interstitiel (ISF) ou le plasma.

10. Agoniste ou antagoniste inverse sélectif des récepteurs de sérotonine 5-HT2A, ou sel pharmaceutiquement acceptable de celui-ci pour utilisation selon l'une quelconque des revendications 1 à 9, où le sujet est identifié par imagerie des plaques amyloïdes au moyen de la tomographie par émission de positons (TEP) du cerveau du sujet ; ou le sujet est identifié par des mutations sur le chromosome 21, des mutations sur le chromosome 14 ou des mutations sur le chromosome 1.

11. Agoniste ou antagoniste inverse sélectif des récepteurs de sérotonine 5-HT2A, ou sel pharmaceutiquement acceptable de celui-ci pour utilisation selon l'une quelconque des revendications 1 à 10, où les peptides Aβ sont choisis dans le groupe constitué de Aβ38, Aβ40, Aβ42 et Aβ43, de préférence de Aβ40 et Aβ42.
